# EUROPEAN PATENT APPLICATION

(11) **EP 1 285 658 A2**
(43) Date of publication of application: **26.02.2003**
(21) Application number: 02255803.5
(22) Date of filing: 20.08.2002
(51) Int. Cl.: A61K 31/519, A61P 15/00

(54) **Treatments for female sexual dysfunction**

(30) Priority: 21.08.2001 US 314230 P
(71) Applicant: Pfizer Productors Inc., Groton, CT 06340 (US)
(72) Inventor: Carpino, Philip Albert, Groton, Connecticut 06340 (US); Hadcock, John Richard Neville, Groton, Connecticut 06340 (US)
(74) Representative: England, Peter Michael

(57) **Abstract**

The present invention provides a method of treating female sexual dysfunction, the method comprising the step of administering to a patent, having or at risk of having one or more of the disorders or conditions associated with female sexual dysfunction, a therapeutically effective amount of a compound that attenuates the binding of agouti-related protein to melanocortin receptors, but does not attenuate the binding of α-melanocyte stimulating hormone to melanocortin receptors. The present invention also provides a method of identifying a compound that is useful for the treatment or prevention of female sexual dysfunction, the method comprising the steps of: 1) determining if a compound affects the binding of agouti-related protein to melanocortin receptors; 2) determining if a compound affects the binding of α-melanocyte stimulating hormone to melanocortin receptors; and 3) selecting a compound that attenuates the binding of agouti-related protein to melanocortin receptors, but does not affect the binding of α-melanocyte stimulating hormone to melanocortin receptors.

## Description

### Field of the Invention

The present invention provides methods for treating the various diseases, disorders and conditions associated with female sexual dysfunction, the methods comprising the step of administering to a female patient in need thereof a therapeutically effective amount of a compound that attenuates the binding of agouti-related protein to melanocortin receptors, but does not attenuate the binding of α-melanocyte stimulating hormone to melanocortin receptors. Optionally, the above methods of the present invention further include melanocortin receptor agonists. Optionally, the above methods may also be used in combination with other compounds useful to treat female sexual dysfunction, such as estrogen agonists /antagonists, cyclic guanosine 3',5'-monophosphate elevator compounds, and estrogens optionally progestins. The present invention is also directed to pharmaceutical compositions and kits containing such compositions.

The present invention also provides methods of identifying compounds that are useful for the treatment of female sexual dysfunction, the methods comprising the steps of: 1) determining if a compound affects the binding of agouti-related protein to melanocortin receptors; 2) determining if a compound affects the binding of α-melanocyte stimulating hormone to melanocortin receptors; and 3) selecting a compound that attenuates the binding of agouti-related protein to melanocortin receptors, but does not attenuate the binding of α-melanocyte stimulating hormone to melanocortin receptors.

### Background of the Invention

Agouti-related protein has been shown to be a potent, selective, endogenous antagonist of melancortin-3 (MCR-3) and melanocortin-4 (MCR-4) receptors, which have been implicated in sexual function (Melanotan II data in human males) and body weight regulation. It has also been shown that ubiquitous expression of human AGRP in transgenic mice results in obesity. In contrast, α-melanocyte stimulating hormone (α-MSH) decreases feeding and is an endogenous agonist of MCR-4 and MCR-3. Additionally the synthetic MCR3/MCR4 agonist MTII is a potent erectogenic agent in humans (see , e.g., H. Wessells et al., "Effect of an Alpha-Melanocyte Stimulating Hormone Analog on Penile Erection and Sexual Desire in Men with Organic Erectile Dysfunction," Urology, 56: 641-646 (2000)). It is believed that the binding sites for α-MSH (and MTII) and AGRP on melanocortin-4 receptors are different, but may partially overlap. Further study has been done on identifying selective human melanocortin-3 receptors (see, e.g., P. Grieco et al., "D-Amino Acid Scan of γ-Melanocyte-Stimulating Hormone: Importance of Trp8 on Human MC3 Receptor Selectivity," J. Med. Chem., 43(26), 4998-5002 (2000).) In addition, U.S. non-provisional patent application, Serial No. 09/761,320, filed January 16, 2001, (which has also published as EP 1125579), discloses treatments for obesity and methods for identifying compounds useful for treating obesity.

In one embodiment, the present invention provides a method of identifying compounds useful to treat female sexual dysfunction, the compounds being selected from compounds that attenuate the binding of agouti-related protein to melanocortin receptors, but do not attenuate the binding of α-melanocyte stimulating hormone to melanocortin receptors.

Female sexual dysfunction (FSD) includes many categories of diseases, conditions and disorders. For example, FSD includes diseases, conditions and disorders, such as the following: hypoactive sexual desire disorder, sexual anhedonia, dyspareunia, sexual arousal disorder and vaginismus.

Proper sexual functioning in women depends on the sexual response cycle, which consists of an anticipatory mental set (sexual motive state or state of desire), effective vasocongestive arousal (swelling and lubrication), orgasm, and resolution. In women, orgasm is accompanied by contractions (not always subjectively experienced as such) of the muscles of the outer third of the vagina. Generalized muscular tension, perineal contractions, and involuntary pelvic thrusting (every 0.8 sec) usually occur. Orgasm is followed by resolution--a sense of general pleasure, well-being, and muscular relaxation. During this phase, women may be able to respond to additional stimulation almost immediately. (For a general review, see C.M. Meston et al., "The neurobiology of sexual function," Arch. Gen. Psychiatry (2000), 57(11), 1012-1030.)

The sexual response cycle is mediated by a delicate, balanced interplay between the sympathetic and parasympathetic nervous systems. Vasocongestion is largely mediated by parasympathetic (cholinergic) outflow; orgasm is predominantly sympathetic (adrenergic). These responses are easily inhibited by cortical influences or by impaired hormonal, neural, or vascular mechanisms. Disorders of sexual response may involve one or more of the cycle's phases. Generally, both the subjective components of desire, arousal, and pleasure and the objective components of performance, vasocongestion, and orgasm are disturbed, although any may be affected independently. Sexual dysfunctions may be lifelong (no effective performance ever, generally due to intrapsychic conflicts) or acquired (after a period of normal function); generalized or limited to certain situations or certain partners; and total or partial.

Hypoactive sexual desire disorder is a disorder in which sexual fantasies and desire for sexual activity are persistently or recurrently diminished or absent, causing marked distress or interpersonal difficulties. Hypoactive sexual desire disorder may be lifelong or acquired, generalized (global) or situational (partner-specific). Sexual desire is a complex psychosomatic process based on brain activity (the "generator" or "motor" running in a rheostatic cyclic fashion), a poorly defined hormonal milieu, and cognitive scripting that includes sexual aspiration and motivation. Desynchronization of these components results in hypoactive sexual desire disorder.

The acquired form of hypoactive sexual desire disorder is commonly caused by boredom or unhappiness in a long-standing relationship, depression (which leads more often to decreased interest in sex than it does to impotence in the male or to inhibited excitement in the female), dependence on alcohol or psychoactive drugs, side effects from prescription drugs (e.g., antihypertensives, antidepressants), and hormonal deficiencies. This disorder can be secondary to impaired sexual functioning in the arousal or orgasm phase of the sexual response cycle.

Symptoms and signs of hypoactive sexual desire disorder include the patient complaining of a lack of interest in sex, even in ordinarily erotic situations. The disorder is usually associated with infrequent sexual activity, often causing serious marital conflict. Some patients have sexual encounters fairly often to please their partners and may have no difficulty with performance but continue to have sexual apathy. When boredom is the cause, frequency of sex with the usual partner decreases, but sexual desire may be normal or even intense with others (the situational form).

Clinically significant sexual dysfunction that causes personal distress or interpersonal problems and is most likely fully explained by direct physiologic effects of a physical disorder. Sexual dysfunction due to a physical disorder is usually generalized (not specific to a given partner or situation). It is diagnosed when evidence from a patient's history, physical examination, or laboratory assessment can explain the dysfunction physiologically and when mental disorders that may better explain it can be ruled out. Resolution of the underlying physical disorders often results in resolution or amelioration of the sexual dysfunction. When the cause of sexual dysfunction is a combination of psychologic and physical factors, the appropriate diagnosis is sexual dysfunction due to combined factors.

Sexual anhedonia (decreased or absent pleasure in sexual activity) is not an official diagnosis. It is almost always classified under hypoactive sexual desire disorder, because loss of pleasure almost always results in loss of desire (although loss of desire may occur first). The cause is likely to be depression or drugs if anhedonia is acquired and global (with all partners in all situations); interpersonal factors if anhedonia is confined to one partner or one situation; or repressive factors (e.g., guilt, shame) due to family dysfunction or childhood trauma if anhedonia is lifelong. Sexual aversion is the probable diagnosis in lifelong cases.

Sexual arousal disorder is the persistent or recurrent inability to attain or to maintain the lubrication-swelling response of sexual excitement until completion of sexual activity. This disturbance occurs despite adequate focus, intensity, and duration of sexual stimulation. The disorder may be lifelong or, more commonly, acquired and restricted to the partner. The patient's complaints are usually related to lack of orgasm, although some women report lack of excitement.

Although women can be orgasmic throughout their lives, sexual activity often decreases after age 60 because of the relative lack of partners and untreated physiologic changes (e.g., atrophy of the vaginal mucosa, with resultant dryness and painful coitus).

The female sexual response phase of arousal is not easily distinguished from the phase of desire until physiological changes begin to take place in the vagina and clitoris as well as other sexual organs. Sexual excitement and pleasure are accompanied by a combination of vascular and neuromuscular events which lead to engorgement of the clitoris, labia and vaginal wall, increased vaginal lubrication and dilatation of the vaginal lumen (see, e.g., Levin, R.J., Clin. Obstet. Gynecol., 1980:7; 213-252; Ottesen, B., Gerstenberg, T., Ulrichsen, H. et al., Eur. J. Clin. Invest., 1983:13; 321-324; Levin, R.J., Exp. Clin. Endocrinol., 1991:98; 61-69; Levin, R.J., Ann. Rev. Sex Res., 1992:3; 1-48; Masters, W. H., Johnson, V. E. Human Sexual Response. Little, Brown: Boston, 1996; Berman, J.R., Berman, L. & Goldstein, L., Urology, 1999:54; 385-391).

Vaginal engorgement enables transudation to occur and this process is responsible for increased vaginal lubrication. Transudation allows a flow of plasma through the epithelium and onto the vaginal surface, the driving force for which is increased blood flow in the vaginal capillary bed during the aroused state. In addition engorgement leads to an increase in vaginal length and luminal diameter, especially in the distal 2/3 of the vaginal canal. The luminal dilatation of the vagina is due to a combination of smooth muscle relaxation of its wall and skeletal muscle relaxation of the pelvic floor muscles. Some sexual pain disorders such as vaginismus are thought to be due, at least in part, by inadequate relaxation preventing dilatation of the vagina; it has yet to be ascertained if this is primarily a smooth or skeletal muscle problem. (see, e.g., Masters, W. H., Johnson, V. E. Human Sexual Response. Little, Brown: Boston, 1996; Berman, J.R., Berman, L. & Goldstein, L., Urology, 1999:54; 385-391).

The categories of FSD are best defined by contrasting them to the phases of normal female sexual response: desire, arousal and orgasm. Desire or libido is the drive for sexual expression. Its manifestations often include sexual thoughts either when in the company of an interested partner or when exposed to other erotic stimuli. Arousal is the vascular response to sexual stimulation, an important component of which is vaginal lubrication and elongation of the vagina. Orgasm is the release of sexual tension that has culminated during arousal.

Hence, FSD occurs when a woman has an inadequate or unsatisfactory response in any of these phases; desire, arousal or orgasm. FSD categories include, for example, hypoactive sexual desire disorder, sexual arousal disorder, orgasmic disorders and sexual pain disorders.

Hypoactive sexual desire disorder is present if a woman has no or little desire to be sexual, and has no or few sexual thoughts or fantasies. This type of FSD can be caused by low testosterone levels, due either to natural menopause or to surgical menopause. Other causes include illness, medications, fatigue, depression and anxiety.

Sexual arousal disorder (FSAD) is characterized by inadequate genital response to sexual stimulation. The genitalia do not undergo the engorgement that characterizes normal sexual arousal. The vaginal walls are poorly lubricated, so that intercourse is painful. Orgasms may be impeded. Arousal disorder can be caused by reduced estrogen at menopause or after childbirth and during lactation, as well as by illnesses, with vascular components such as diabetes and atherosclerosis. Other causes result from treatment with diuretics, antihistamines, antidepressants, e.g., SSRIs or antihypertensive agents.

Sexual pain disorders (includes dyspareunia and vaginismus) is characterized by pain resulting from penetration and may be caused by medications which reduce lubrication, endometriosis, pelvic inflammatory disease, inflammatory bowel disease or urinary tract problems.

Dyspareunia is painful coitus or attempted coitus. Dyspareunia is usually introital but may also occur before, during, or after intercourse. Causes include menopausal involution with dryness and thinning of the mucosa. Pain during or after coitus is the chief complaint.

The prevalence of FSD is difficult to gauge because the term covers several types of problems, some of which are difficult to measure, and because the interest in treating FSD is relatively recent. Many women's sexual problems are associated either directly with the female aging process or with chronic illnesses such as diabetes or hypertension.

There are wide variations in the reported incidence and prevalence of FSD, in part explained by the use of differing evaluation criteria, but most investigators report that a significant proportion of otherwise healthy women have symptoms of one or more of the FSD subgroups. By way of example, studies comparing sexual dysfunction in couples reveal that 63% of women had arousal or orgasmic dysfunction compared with 40% of men having erectile or ejaculatory dysfunction (see, e.g., Frank, E., Anderson, C. & Rubinstein, D., N. Engl. J. Med., 11:229;111-115). However, the prevalence of female sexual arousal disorder varies considerably from survey to survey. In a recent National Health and Social Life Survey, 19% of women reported lubrication difficulties whereas 14% of women in an outpatient gynecological clinic reported similar difficulties with lubrication (Rosen, R., Taylor, J., Leiblum, S. et al., J. Sex Marital Ther., 1993:19; 171-188).

Several studies have also reported dysfunction with sexual arousal in diabetic women (up to 47%), including reduced vaginal lubrication (Wincze, J.P., Albert, A. & Bansal, S., Arch. Sex Behav., 1993:22; 587-601). There was no association between neuropathy and sexual dysfunction. Numerous studies have also shown that between 11-48% of women overall may have reduced sexual desire with age. Similarly, between 11-50% of women report problems with arousal and lubrication, and therefore experience pain with intercourse. Vaginismus is far less common, affecting approximately 1 % of women. Studies of sexually experienced women have detailed that 5-10% have primary anorgasmia. Another 10% have infrequent orgasms and a further 10% experience them inconsistently (Spector, I.P. & Carey, M.P., Arch. Sex. Behav., 1990:19; 389-408).

FSAD is a highly prevalent sexual disorder affecting pre-, peri- and post menopausal (±HRT) women. It is associated with concomitant disorders such as depression, cardiovascular diseases, diabetes and UG disorders. The primary consequences of FSAD are lack of engorgement/swelling, lack of lubrication and lack of pleasurable genital sensation. The secondary consequences of FSAD are reduced sexual desire, pain during intercourse and difficulty in achieving an orgasm. It has recently been hypothesized that there is a vascular basis for at least a proportion of patients with symptoms of FSAD (Goldstein, L. & Berman, J.R., Int. J. Impot. Res., 1998:10; S84-S90) with animal data supporting this view (Park, K., Goldstein, I., Andry, C., et al., Int. J. Impotence Res., 1997:9; 27-37).

In accordance with the teachings herein, compounds identified by the present invention that attenuate the binding of agouti-related protein to melanocortin receptors, but do not attenuate the binding of α-melanocyte stimulating hormone to melanocortin receptors are useful to treat female sexual dysfunction.

The hormone estrogen has a profound effect in the vascular system of both men and women although its administration is associated with other effects that can be undesirable. Estrogen increases vasodilatation and inhibits the response of blood vessels to injury and the development of atherosclerosis. Estrogen-induced vasodilatation occurs 5 to 20 minutes after estrogen has been administered and is not dependent on changes in gene expression; this action of estrogen is sometimes referred to as "nongenomic." The estrogen-induced inhibition of the response to vascular injury and the preventive effect of estrogen against atherosclerosis occur over a period of hours or days after estrogen treatment and are dependent on changes in gene expression in the vascular tissues; these actions are sometimes referred to as "genomic."

There are two estrogen receptors, estrogen receptor α and estrogen receptor β, both of which are members of the superfamily of steroid hormone receptors. (Walter P., et al., Proc Nad Acad Sci USA 1985;82:7889-93; Kuiper G.G.J.M., et al., Proc Nad Acad Sci USA 1996;93:5925-30) Estrogen receptors α and β have considerable homology and, like all steroid hormone receptors, are transcription factors that alter gene expression when they are activated. (Walter P., et al., Proc Nad Acad Sci USA 1985;82:7889-93; Kuiper G.G.J.M., et al., Proc Nad Acad Sci USA 1996;93:5925-30; Shibata H., et al., Recent Prog Horm Res 1997;52:141-65; Evans R.M., Science 1988;240:889-95; Brown M., Hematol Oncol Clin North Am 1994;8:101-12). Blood vessels are complex structures, with walls containing smooth-muscle cells and an endothelial cell lining. Vascular endothelial and smooth muscle cells bind estrogen with high affinity (Mendelsohn M.E., et al., Curr Opin Cardiol 1994;9:619-26; Farhat M.Y., et al., FASEB J 1996;10:615-24) and estrogen receptor α has been identified in both types of vascular cells in women and men, (Karas R.H., et al., Circulation 1994;89:1943-50; Losordo D.W., et al., Circulation 1994;89:1501-10; Venkov C.D., et al., Circulation 1996;94:727-33; Kim-Schulze S., et al., Circulation 1996;94:1402-7; Caulin-Glaser T., et al., J Clin Invest 1996;98:36-42) as well as in myocardial cells (Grohe C., et al., FEBS Lett 1997;416:107-12).

Estrogen receptor α activates specific target genes in vascular smooth-muscle and endothelial cells (Karas R.H., et al., Circulation 1994;89:1943-50, Venkov C.D., et al., Circulation 1996;94:727-33; Kim-Schulze S., et al., Circulation 1996;94:1402-7; Caulin-Glaser T., et al., J Clin Invest 1996;98:36-42; Koike H., et al., J Vase Surg 1996;23:477-82). Estrogen receptor β is structurally and functionally distinct from estrogen receptor α. Functional estrogen receptor β is also present in myocardial cells, in which it regulates the expression of nitric oxide synthases.

In premenopausal women, 17β-estradiol produced by the ovaries is the chief circulating estrogen. Serum estradiol concentrations are low in preadolescent girls and increase at menarche. In women, they range from about 100 pg per milliliter (367 pmol per liter) in the follicular phase to about 600 pg per milliliter (2200 pmol per liter) at the time of ovulation. They may rise to nearly 20,000 pg per milliliter (70,000 pmol per liter) during pregnancy. After menopause, serum estradiol concentrations fall to values similar to or lower than those in men of similar age (5 to 20 pg per milliliter [18 to 74 pmol per liter]) (Yen, S.S.C. and Jaffe, R.B. eds., Reproductive Endocrinology: Physiology, Pathophysiology and Clinical Management, 3rd ed. Philadelphia: W.B. Saunders, 1991).

While estrogenic effects can provide vascular benefits and prevent and reverse vaginal atrophy in postmenopausal women, the administration of estrogen alone can have deleterious effects. For example, breast cancer is a hormone-dependent disease. Women without functioning ovaries who never receive estrogen replacement do not develop breast cancer. The female-to-male ratio for the disease is about 150 to 1. A host of findings indicate that hormones play a critical role as promoters of the disease. For most epithelial malignancies, a log-log plot of incidence versus age shows a straight-line increase with every year of life. A similar plot for breast cancer shows the same straight-line increase, but with a decrease in slope beginning at the age of menopause. The three dates in a woman's life that have a major impact on breast cancer incidence are age of menarche, age at first full-term pregnancy, and age of menopause. Women who experience menarche at age 16 have only 50 to 60 percent of the lifetime breast cancer risk of women who experience menarche at age 12. Similarly, menopause occurring 10 years before the median age (52 years), whether natural or surgically induced, reduces lifetime breast cancer risk by about 35 percent. Compared with nulliparous women, women who have a first full-term pregnancy by age 18 have 30 to 40 percent the risk of breast cancer. Thus, length of menstrual life--particularly the fraction occurring before the first full-term pregnancy--is a substantial component of the total risk of breast cancer. This factor can account for 70 to 80 percent of the variation in breast cancer frequency in different countries.

International variation has provided some of the most important clues on hormonal carcinogenesis. A woman living to age 80 in North America has 1 chance in 9 of developing invasive breast cancer. Asian women have one-fifth to one-tenth the risk of breast cancer of women in North America or Western Europe. Asian women have substantially lower concentrations of estrogens and progesterone. These differences cannot be explained on a genetic basis, because Asian women living in a Western environment have a risk identical to that of their Western counterparts. These women also differ markedly in height and weight from Asian women in Asia; height and weight are critical regulators of age of menarche and have substantial effects on plasma concentrations of estrogens. (Lippman, M.E., *Breast Cancer,* Chapter 91, in Harrison's Principles of Internal Medicine, 14th ed., 1998). Thus despite the beneficial effects which estrogens play in maintaining health, the administration of estrogens may also cause adverse effects on a subject's health such as an increased risk of breast cancer.

Menopause occurs naturally at an average age of 50 to 51 years in the USA. As ovaries age, response to pituitary gonadotropins (follicle-stimulating hormone [FSH] and luteinizing hormone [LH]) decreases, initially resulting in shorter follicular phases (thus, shorter menstrual cycles), fewer ovulations, decreased progesterone production, and more irregularity in cycles. Eventually, the follicle fails to respond and does not produce estrogen. The transitional phase, during which a woman passes out of the reproductive stage, begins before menopause. It is termed the climacteric or perimenopause, although many persons refer to it as menopause.

Premature menopause refers to ovarian failure of unknown cause that occurs before age 40. It may be associated with smoking, living at high altitude, or poor nutritional status. Artificial menopause may result from oophorectomy, chemotherapy, radiation of the pelvis, or any process that impairs ovarian blood supply.

The compounds identified by the present invention act to treat female sexual dysfunction. Such compounds may be administered either singly or in combination with other agents useful to treat female sexual dysfunction, such as estrogen agonists / antagonists, as described further below.

Also, for the treatment of female subject sexual dysfunction, the compounds identified by the present invention can be administered either singly or in combination with agents that elevate cyclic guanosine 3',5'-monophosphate (cGMP). Agents that elevate cGMP levels are well known and can work through any of several mechanisms. Agents which selectively inhibit an enzyme predominantly involved in cGMP breakdown, for example a cGMP-dependent phosphodiesterase, constitute one example.

In particular, cyclic guanosine 3',5'-monophosphate phosphodiesterase (cGMP PDE) inhibitors are widely known as cardiovascular agents for the treatment of conditions such as angina, hypertension, and congestive heart failure. More recently cGMP PDE inhibitors capable of inhibiting type V phosphodiesterase (cGMP PDE_{V}) have been found to be effective for the treatment of male erectile dysfunction, importantly by oral administration. See, for example, PCT/EP94/01580, published as WO 94/28902 which designates, *inter alia,* the United States.

### Brief Description of the Drawing

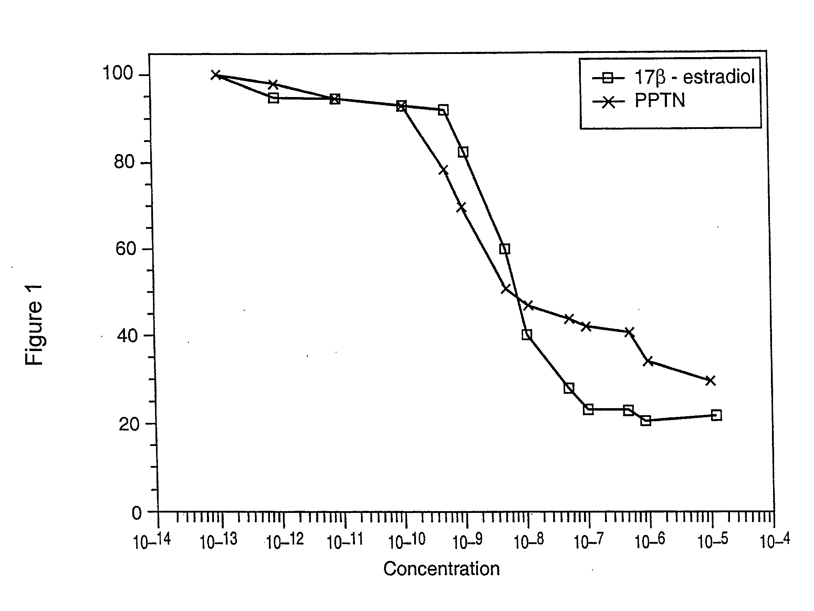

Figure 1 is a log-linear competition binding plot of PPTN and 17β-estradiol to human estrogen receptor. The X-axis represents percentage of radiolabeled estrogen bound to receptor. The Y-axis represents molar concentration of added ligand. Values are mean ± SEM.

### Summary of the Invention

The present invention provides methods of treating female sexual dysfunction, the methods comprising the step of administering to a female patient in need thereof a therapeutically effective amount of a compound that attenuates the binding of agouti-related protein to melanocortin receptors, but does not attenuate the binding of α-melanocyte stimulating hormone to melanocortin receptors.

The present invention also provides methods of treating female sexual dysfunction, the methods comprising the step of administering to a female patient in need thereof a therapeutically effective amount of a compound that attenuates the binding of agouti-related protein to melanocortin receptors, but does not attenuate the binding of α-melanocyte stimulating hormone to melanocortin receptors in combination with a compound that is a melanocortin receptor agonist.

In a preferred embodiment of the methods of treating female sexual dysfunction, the female sexual dysfunction is other than hypoactive sexual desire disorder, sexual anhedonia or dyspareunia.

The present invention provides methods of treating sexual arousal disorder in a female patient which comprises administering to a female patient in need thereof a therapeutically effective amount of a compound that attenuates the binding of agouti-related protein to melanocortin receptors, but does not attenuate the binding of α-melanocyte stimulating hormone to melanocortin receptors.

The present invention provides methods of treating vaginismus in a female patient which comprises administering to a female patient in need thereof a therapeutically effective amount of a compound that attenuates the binding of agouti-related protein to melanocortin receptors, but does not attenuate the binding of α-melanocyte stimulating hormone to melanocortin receptors

The present invention provides methods of increasing the frequency or intensity of orgasms in a female patient which comprises administering to a female patient in need thereof a therapeutically effective amount of a compound that attenuates the binding of agouti-related protein to melanocortin receptors, but does not attenuate the binding of α-melanocyte stimulating hormone to melanocortin receptors.

The present invention provides methods of enhancing libido, preferably more than normal, in a female patient which comprises administering to a female patient in need thereof a therapeutically effective amount of a compound that attenuates the binding of agouti-related protein to melanocortin receptors, but does not attenuate the binding of α-melanocyte stimulating hormone to melanocortin receptors.

In a preferred embodiment of the methods of the present invention, the melanocortin receptors are melanocortin-3 and/or 4 receptors.

In a more preferred embodiment of the methods of the present invention, the melanocortin receptors are melanocortin-4 receptors.

Another aspect of the present invention provides the above methods comprising the administration of the above compounds and the co-administration of a therapeutically effective amount of an estrogen agonist / antagonist.

Another aspect of the present invention provides the above methods comprising the administration of the above compounds and the co-administration of a therapeutically effective amount of a cyclic guanosine 3',5'-monophosphate elevator.

In a preferred embodiment of the present invention, the patient is a female patient. For example, the female patient may be a postmenopausal female subject.

Another aspect of the present invention provides the above methods comprising the administration of the above compounds and the co-administration of a therapeutically effective amount of an estrogen optionally with a progestin.

Another aspect of the present invention provides the above methods comprising the administration of the above compounds and the co-administration of a therapeutically effective amount of a compound selected from the group consisting of: Prostaglandins; Apomorphine; Oxytocin modulators; α-2 Adrenergic antagonists; Androgens; selective androgen receptor modulators (SARMs); bupropion; Vasoactive intestinal peptide (VIP); Neutral endopeptidase inhibitors (NEP); and Neuropeptide Y receptor antagonists (NPY).

The present invention also provides methods of identifying a compound that is useful for the treatment of female sexual dysfunction, the methods comprising the steps of:
1) determining if a compound affects the binding of agouti-related protein to melanocortin receptors;
2) determining if a compound affects the binding of α-melanocyte stimulating hormone to melanocortin receptors; and
3) selecting a compound that attenuates the binding of agouti-related protein to melanocortin receptors, but does not attenuate the binding of α-melanocyte stimulating hormone to melanocortin receptors.

In a preferred embodiment of the methods of identifying a compound, the determination of whether a compound affects the binding of agouti-related protein to melanocortin receptors is accomplished using a competitive binding assay.

In a preferred embodiment of the methods of identifying a compound, the determination of whether a compound affects the binding of α-melanocyte stimulating hormone to melanocortin receptors is accomplished using a competitive binding assay.

In a more preferred embodiment of the methods of identifying a compound, the determination of whether a compound affects the binding of agouti-related protein to melanocortin receptors is accomplished using a competitive binding assay, and the determination of whether compounds affects the binding of α-melanocyte stimulating hormone to melanocortin receptors is accomplished using a competitive binding assay.

In a preferred embodiment of the methods of identifying a compound, the melanocortin receptors are melanocortin-3 and/or melanocortin-4 receptors.

In a more preferred embodiment of the methods of identifying a compound, the melanocortin receptors are melanocortin-4 receptors.

The present invention also provides pharmaceutical compositions for treating female sexual dysfunction that comprise a compound that attenuates the binding of agouti-related protein to melanocortin receptors, but does not attenuate the binding of α-melanocyte stimulating hormone to melanocortin receptors.

The present invention also provides pharmaceutical compositions for treating female sexual dysfunction that comprise 1) a compound that attenuates the binding of agouti-related protein to melanocortin receptors, but does not attenuate the binding of α-melanocyte stimulating hormone to melanocortin receptors and 2) a compound that is a melanocortin receptor agonist.

The present invention also provides pharmaceutical compositions that comprise 1) a compound that attenuates the binding of agouti-related protein to melanocortin receptors, but does not attenuate the binding of α-melanocyte stimulating hormone to melanocortin receptors, which compound is useful to treat female sexual dysfunction; 2) a compound that is a melanocortin receptor agonist; and 3) a second compound useful for the treatment of female sexual dysfunction.

The present invention also provides pharmaceutical compositions that comprise 1) a compound that attenuates the binding of agouti-related protein to melanocortin receptors, but does not attenuate the binding of α-melanocyte stimulating hormone to melanocortin receptors, which compound is useful to treat female sexual dysfunction; and 2) a second compound useful for the treatment of female sexual dysfunction.

The present invention also provides kits for the treatment of female sexual dysfunction, the kits comprising:
a) a first pharmaceutical composition comprising a compound that attenuates the binding of agouti-related protein to melanocortin receptors, but does not attenuate the binding of α-melanocyte stimulating hormone to melanocortin receptors;
b) a second pharmaceutical composition comprising a second compound useful for the treatment of female sexual dysfunction; and
c) a container for the first and second compositions.

Another aspect of the present invention, the present invention provides for kits for use by a consumer and, preferably, a postmenopausal female subject to treat female sexual dysfunction. The kits comprise: a) a first pharmaceutical composition comprising a compound that attenuates the binding of agouti-related protein to melanocortin receptors, but does not attenuate the binding of α-melanocyte stimulating hormone to melanocortin receptors; and a pharmaceutically acceptable carrier, vehicle or diluent; and optionally, b) a second pharmaceutical composition comprising an estrogen agonist / antagonist and a pharmaceutically acceptable carrier, vehicle or diluent; and optionally, c) instructions describing a method of using the pharmaceutical compositions for treating female sexual dysfunction. When the kit comprises a compound of the present invention and an estrogen agonist / antagonist, they may be optionally combined in the same pharmaceutical composition.

Additional kits comprise: a) a first pharmaceutical composition comprising a compound that attenuates the binding of agouti-related protein to melanocortin receptors, but does not attenuate the binding of α-melanocyte stimulating hormone to melanocortin receptors; and a pharmaceutically acceptable carrier, vehicle or diluent; and optionally, b) a second pharmaceutical composition comprising a cGMP elevator and a pharmaceutically acceptable carrier, vehicle or diluent; and optionally, c) instructions describing a method of using the pharmaceutical compositions for treating female sexual dysfunction. When the kit comprises a compound of the present invention and a cGMP elevator, they may be optionally combined in the same pharmaceutical composition.

The present invention also provides the following additional pharmaceutical compositions and kits, such as the following:

A pharmaceutical composition which comprises 1) a compound that attenuates the binding of agouti-related protein to melanocortin receptors, but does not attenuate the binding of α-melanocyte stimulating hormone to melanocortin receptors, and 2) an estrogen agonist / antagonist or a pharmaceutically acceptable salt thereof.

A pharmaceutical composition which comprises 1) a compound that attenuates the binding of agouti-related protein to melanocortin receptors, but does not attenuate the binding of α-melanocyte stimulating hormone to melanocortin receptors; 2) a compound that is a melanocortin receptor agonist; and 3) an estrogen agonist / antagonist or a pharmaceutically acceptable salt thereof.

In a preferred embodiment of these compositions, said estrogen agonist / antagonist is (-)-cis-6-phenyl-5-[4-(2-pyrrolidin-1-yl-ethoxy)-phenyl]-5,6,7,8-tetrahydro-naphthalene-2-ol (also known as lasofoxifene) or an optical or geometric isomer thereof; a pharmaceutically acceptable salt, N-oxide, ester, quaternary ammonium salt, or a prodrug thereof. More preferably, said estrogen agonist / antagonist is in the form of a D-tartrate salt.

In a preferred embodiment of these compositions, said estrogen agonist / antagonist is selected from the group consisting of tamoxifen, 4-hydroxy tamoxifen, raloxifene, droloxifene, toremifene, centchroman, idoxifene, 6-(4-hydroxy-phenyl)-5-[4-(2-piperidin-1-yl-ethoxy)-benzyl]-naphthalen-2-ol, {4-[2-(2-aza-bicyclo[2.2.1]hept-2-yl)-ethoxy]-phenyl}-[6-hydroxy-2-(4-hydroxy-phenyl)-benzo[b]thiophen-3-yl]-methanone, EM-652, EM-800, GW 5638, GW 7604, TSE-424 and optical or geometric isomers thereof; and pharmaceutically acceptable salts, N-oxides, esters, quaternary ammonium salts, and prodrugs thereof.

A pharmaceutical composition for treating female sexual dysfunction which comprises 1) a compound that attenuates the binding of agouti-related protein to melanocortin receptors, but does not attenuate the binding of α-melanocyte stimulating hormone to melanocortin receptors, and 2) a compound selected from the group consisting of a cyclic guanosine 3',5'-monophosphate elevator. In a preferred embodiment of such compositions, said cyclic guanosine 3',5'-monophosphate elevator is a PDE_{V} phosphodiesterase inhibitor. In a more preferred embodiment, said PDE_{V} phosphodiesterase inhibitor is 1-[[3-(6,7-dihydro-1-methyl-7-oxo-3-propyl-1 H-pyrazolo[4,3-d]pyrimidin-5-yl)-4-ethoxy-phenyl]sufonyl]-4-methylpiperazine citrate salt.

A pharmaceutical composition which comprises 1) a compound that attenuates the binding of agouti-related protein to melanocortin receptors, but does not attenuate the binding of α-melanocyte stimulating hormone to melanocortin receptors, and 2) an estrogen optionally with a progestin. In a preferred embodiment of such compositions, the estrogen is Premarin®.

A pharmaceutical composition which comprises 1) a compound that attenuates the binding of agouti-related protein to melanocortin receptors, but does not attenuate the binding of α-melanocyte stimulating hormone to melanocortin receptors, and 2) a compound selected from the group consisting of Prostaglandins; Apomorphine; Oxytocin modulators; α-2 Adrenergic antagonists; Androgens; selective androgen receptor modulators (SARMs); bupropion; Vasoactive intestinal peptide (VIP); Neutral endopeptidase inhibitors (NEP); and Neuropeptide Y receptor antagonists (NPY).

A pharmaceutical composition for treating female sexual dysfunction which comprises 1) a compound that attenuates the binding of agouti-related protein to melanocortin receptors, but does not attenuate the binding of α-melanocyte stimulating hormone to melanocortin receptors; 2) a compound that is a melanocortin receptor agonist; and 3) a compound selected from the group consisting of a cyclic guanosine 3',5'-monophosphate elevator. In a preferred embodiment, said cyclic guanosine 3',5'-monophosphate elevator is a PDE_{V} phosphodiesterase inhibitor. In a more preferred embodiment, said PDE_{V} phosphodiesterase inhibitor is 1-[[3-(6,7-dihydro-1-methyl-7-oxo-3-propyl-1H-pyrazolo[4,3-d]pyrimidin-5-yl)-4-ethoxy-phenyl]sufonyl]-4-methylpiperazine citrate salt.

A pharmaceutical composition which comprises 1) a compound that attenuates the binding of agouti-related protein to melanocortin receptors, but does not attenuate the binding of α-melanocyte stimulating hormone to melanocortin receptors; 2) a compound that is a melanocortin receptor agonist; and 3) an estrogen optionally with a progestin. In a preferred embodiment, the estrogen is Premarin®.

A pharmaceutical composition which comprises 1) a compound that attenuates the binding of agouti-related protein to melanocortin receptors, but does not attenuate the binding of α-melanocyte stimulating hormone to melanocortin receptors; 2) a compound that is a melanocortin receptor agonist; and 3) a compound selected from the group consisting of Prostaglandins; Apomorphine; Oxytocin modulators; α-2 Adrenergic antagonists; Androgens; selective androgen receptor modulators (SARMs); bupropion; Vasoactive intestinal peptide (VIP); Neutral endopeptidase inhibitors (NEP); and Neuropeptide Y receptor antagonists (NPY).

A pharmaceutical composition that comprises 1) a compound that attenuates the binding of agouti-related protein to melanocortin receptors, but does not attenuate the binding of α-melanocyte stimulating hormone to melanocortin receptors, which compound is useful to treat sexual arousal disorder, treat vaginismus, enhance libido more than normal or increase the frequency or intensity of orgasms; 2) a compound that is a melanocortin receptor agonist; and 3) a second compound useful to treat sexual arousal disorder, treat vaginismus, enhance libido more than normal or increase the frequency or intensity of orgasms.

A pharmaceutical composition that comprises 1) a compound that attenuates the binding of agouti-related protein to melanocortin receptors, but does not attenuate the binding of α-melanocyte stimulating hormone to melanocortin receptors, which compound is useful to treat sexual arousal disorder, treat vaginismus, enhance libido more than normal or increase the frequency or intensity of orgasm ; and 2) a second compound useful to treat sexual arousal disorder, treat vaginismus, enhance libido more than normal or increase the frequency or intensity of orgasms.

A kit to treat sexual arousal disorder, treat vaginismus, enhance libido more than normal or increase the frequency or intensity of orgasms comprising:
a) a first pharmaceutical composition comprising a compound that attenuates the binding of agouti-related protein to melanocortin receptors, but does not attenuate the binding of α-melanocyte stimulating hormone to melanocortin receptors;
b) a second pharmaceutical composition comprising a second compound useful to treat sexual arousal disorder, treat vaginismus, enhance libido more than normal or increase the frequency or intensity of orgasms;
c) a container for the first and second compositions.

### Detailed Description of the Invention

The present invention provides methods of treating female sexual dysfunction, the methods comprising the step of administering to a female patent in need thereof a therapeutically effective amount of a compound that attenuates the binding of agouti-related protein to melanocortin receptors, but does not attenuate the binding of α-melanocyte stimulating hormone to melanocortin receptors. Preferably, the receptors are melanocortin-3 and/or melanocortin-4 receptors. More preferably, the receptors are melanocortin-4 receptors.

The present invention also provides methods of identifying a compound that is useful for the treatment of female sexual dysfunction, the methods comprising the steps of: 1) determining if a compound affects the binding of agouti-related protein to melanocortin receptors; 2) determining if a compound affects the binding of α-melanocyte stimulating hormone to melanocortin receptors; and 3) selecting a compound that attenuates the binding of agouti-related protein to melanocortin receptors, but does not attenuate the binding of α-melanocyte stimulating hormone to melanocortin receptors. In a preferred embodiment of the methods, the melanocortin receptors are melanocortin-3 and/or melanocortin-4 receptors. More preferably, the receptors are melanocortin-4 receptors.

The term "therapeutically effective amount" means an amount of a compound or combination of compounds that ameliorates, attenuates, or eliminates one or more symptoms of a particular disease or condition or prevents or delays the onset of one of more symptoms of a particular disease or condition.

The term "patient" means animals, such as dogs, cats, cows, horses, sheep, geese, and humans. Particularly preferred patients are mammals, including both males and females.

The term "pharmaceutically acceptable" means that the substance or composition must be compatible with the other ingredients of a formulation, and not deleterious to the patient.

The term "postmenopausal women" is defined to include not only women of advanced age who have passed through menopause, but also women who have been hysterectomized or for some other reason have suppressed estrogen production, such as those who have undergone long-term administration of corticosteroids, suffer from Cushions' syndrome or have gonadal dysgenesis.

The term "attenuates" with regard to inhibition of AGRP or α-MSH binding means that the compound prevents the binding of either AGRP or α-MSH to melanocortin receptors or decreases the binding affinity of AGRP to melanocortin receptors. In the case of attenuation of AGRP binding, it is preferable if the compound being tested inhibits 25% of AGRP binding. More preferably, the compound inhibits 50%, and most preferably, greater than 75% of AGRP binding to melanocortin receptors. Similarly, with respect to α-MSH binding to melanocortin receptors, a preferred compound blocks no more than 50% of α-MSH binding. More preferably, the compound blocks no more that 25% of α-MSH binding. In a more preferred embodiment, the compound being tested blocks more than 75% of AGRP binding and blocks less than 25% of α-MSH binding. The percent inhibition of binding can be easily determined by those skilled in the art by competition and other inhibition assays in view of this disclosure. The blockade can be competitive, non-competitive, uncompetitive or a combination. In a preferred embodiment, the attenuation of binding is measured in relation to MCR-3 and/or MCR-4, and more preferably MCR-4.

The terms "reaction-inert solvent" or "inert solvent" refer to a solvent or mixture of solvents that does not interact with starting materials, reagents, intermediates or products in a manner that adversely affects the desired product.

The terms "treating", "treat" or "treatment" include preventative (e.g., prophylactic) and palliative treatment.

The phrase "compound identified by the present invention" and grammatical variations thereof means a compound that attenuates the binding of agouti-related protein to melanocortin receptors, but does not attenuate the binding of α-melanocyte stimulating hormone to melanocortin receptors, or a stereoisomer of the compound, a pharmaceutically acceptable salt of the compound, a prodrug of the compound, or a pharmaceutically acceptable salt of the prodrug. It is also contemplated that any additional pharmaceutically active compound used in combination with a compound identified by the present invention can be a stereoisomer of the additional active compound, a salt of the additional active compound, a prodrug of the additional compound or a salt of the prodrug.

The phrase "a compound that attenuates the binding of agouti-related protein to melanocortin receptors, but does not attenuate the binding of α-melanocyte stimulating hormone to melanocortin receptors" includes the stereoisomers of the compound, salts of the compound, prodrugs of the compound, and salts of the prodrugs.

The characteristics of patients at risk of having female sexual dysfunction are well known to those in the art and include patients who have a family history of cardiovascular disease, including hypertension and atherosclerosis, obese patients, patients who exercise infrequently, patients with hypercholesterolemia, hyperlipidemia and/or hypertriglyceridemia, patients having high levels of LDL or Lp(a), patients having low levels of HDL (hypoalphalipoproteinemia), and the like.

The terms pharmaceutically acceptable salts or prodrugs means the salts and prodrugs of compounds that are, within the scope of sound medical judgment, suitable for use with patients without undue toxicity, irritation, allergic response, and the like, commensurate with a reasonable benefit/risk ratio, and effective for their intended use, as well as the zwitterionic forms, where possible, of the compounds of the present invention.

The term "salts" refers to inorganic and organic salts of compounds. These salts can be prepared in situ during the final isolation and purification of a compound, or by separately reacting a purified compound in its free base form with a suitable organic or inorganic acid and isolating the salt thus formed. Representative salts include the hydrobromide, hydrochloride, sulfate, bisulfate, nitrate, acetate, oxalate, palmitiate, stearate, laurate, borate, benzoate, lactate, phosphate, tosylate, besylate, esylate, citrate, maleate, fumarate, succinate, tartrate, naphthylate, mesylate, glucoheptonate, lactobionate, and laurylsulphonate salts, and the like. These may include cations based on the alkali and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. See, for example, S.M. Berge, et al., "Pharmaceutical Salts," *J Pharm Sci,* **66:**1-19 (1977).

The term "prodrug" means compounds that are transformed *in vivo* to yield an active compound. The transformation may occur by various mechanisms, such as through hydrolysis in blood. A discussion of the use of prodrugs is provided by T. Higuchi and W. Stella, "Pro-drugs as Novel Delivery Systems," Vol. 14 of the *A.C.S. Symposium Series,* and in *Bioreversible Carriers in Drug Design,* ed. Edward B. Roche, American Pharmaceutical Association and Pergamon Press, 1987.

For example, if an active compound contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if a compound comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a compound comprises an amine functional group, a prodrug can be formed by the replacement of a hydrogen atom in the amine group with a group such as R-carbonyl, RO-carbonyl, NRR'-carbonyl where R and R' are each independently ((C₁-C₁₀)alkyl, (C₃-C₇)cycloalkyl, benzyl, or R-carbonyl is a natural α-aminoacyl or natural α-aminoacyl-natural α-aminoacyl, -C(OH)C(O)OY wherein (Y is H, (C₁-C₆)alkyl or benzyl), -C(OY₀)Y₁ wherein Y₀ is (C₁-C₄) alkyl and Y₁ is ((C₁-C₆)alkyl, carboxy(C₁-C₆)alkyl, amino(C₁-C₄)alkyl or mono-N- or di-N,N-(C₁-C₆)alkylaminoalkyl,-C(Y₂)Y₃ wherein Y₂ is H or methyl and Y₃ is mono-N- or di-N,N-(C₁-C₆)alkylamino, morpholino, piperidin-1-yl or pyrrolidin-1-yl.

The compounds identified by the present invention may contain asymmetric or chiral centers, and therefore, exist in different stereoisomeric forms. It is contemplated that all stereoisomeric forms of the compounds as well as mixtures thereof, including racemic mixtures, form part of the present invention. In addition, the present invention contemplates all geometric and positional isomers. For example, if a compound contains a double bond, both the cis and trans forms, as well as mixtures, are contemplated.

Mixtures of isomers, including stereoisomers can be separated into their individual isomers on the basis of their physical chemical differences by methods well know to those skilled in the art, such as by chromatography and/or fractional crystallization. Enantiomers can be separated by converting the enantiomeric mixture into a diasteromeric mixture by reaction with an appropriate optically active compound (e.g., alcohol), separating the diastereomers and converting (e.g., hydrolyzing) the individual diastereomers to the corresponding pure enantiomers. Also, some of the compounds of this invention may be atropisomers (e.g., substituted biaryls) and are considered as part of this invention.

The compounds identified by the present invention may exist in unsolvated as well as solvated forms with pharmaceutically acceptable solvents such as water, ethanol, and the like. The present invention contemplates and encompasses both the solvated and unsolvated forms.

It is also possible that compounds identified by the present invention may exist in different tautomeric forms. All tautomers of compounds of the present invention are contemplated. For example, all of the tautomeric forms of the imidazole moiety are included in this invention. Also, for example, all keto-enol or imine-enamine forms of the compounds are included in this invention.

Those skilled in the art will recognize that compound names contained herein may be based on a particular tautomer of a compound. While the name for only a particular tautomer may be used, it is intended that all tautomers are encompassed by the name of the particular tautomer and all tautomers are considered part of the present invention.

It is also intended that the invention disclosed herein encompass compounds that are synthesized *in vitro* using laboratory techniques, such as those well known to synthetic chemists; or synthesized using *in vivo* techniques, such as through metabolism, fermentation, digestion, and the like. It is also contemplated that compounds may be synthesized using a combination of *in vitro* and *in vivo* techniques.

The present invention also includes isotopically-labelled compounds, which are identical to those recited herein, but for the fact that one or more atoms are replaced by an atom having an atomic mass or mass number different from the atomic mass or mass number usually found most abundantly in nature. Examples of isotopes that can be incorporated into compounds identified by the present invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, ¹³⁵I and ³⁶CI, respectively. Compounds identified by the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labelled compounds can generally be prepared by substituting a readily available isotopically labelled reagent for a non-isotopically labelled reagent.

The compounds identified by the present invention are administered to a patient in a therapeutically effective amount. The compounds can be administered alone or as part of a pharmaceutically acceptable composition. In addition, the compounds or compositions can be administered all at once, as for example, by a bolus injection, multiple times, such as by a series of tablets, or delivered substantially uniformly over a period of time, as for example, using transdermal delivery. It is also noted that the dose of the compound can be varied over time.

In addition, the compounds identified by the present invention can be administered alone, in combination with other compounds identified by the present invention, or with other pharmaceutically active compounds. The other pharmaceutically active compounds can be intended to treat the same disease or condition as the compounds identified by the present invention or different diseases or conditions. If the patient is to receive or is receiving multiple pharmaceutically active compounds, the compounds can be administered simultaneously, or sequentially in any order. For example, in the case of tablets, the active compounds may be found in one tablet or in separate tablets, which can be administered at once or sequentially in any order. In addition, it should be recognized that the compositions may be different forms. For example, one or more compounds may be delivered via a tablet, while another is administered via injection or orally as a syrup. All combinations, delivery methods and administration sequences are contemplated.

For example, "co-administration" of a combination of a compound identified by the present invention and an estrogen agonist / antagonist, a cGMP elevator or other active agents means that these components can be administered together as a composition or as part of the same, unitary dosage form. "Co-administration" also includes administering a compound identified by the present invention and an estrogen agonist / antagonist, a cGMP elevator and other active agents separately but as part of the same therapeutic treatment program or regimen. The components need not necessarily be administered at essentially the same time, although they can if so desired. Thus "co-administration" includes, for example, administering a compound identified by the present invention and an estrogen agonist / antagonist, a cGMP elevator or other active agent as separate dosages or dosage forms, but at the same time. "Co-administration" also includes separate administration at different times and in any order. For example, where appropriate a patient may take one or more component(s) of the treatment in the morning and the one or more of the other component(s) at night.

Since one aspect of the present invention contemplates the treatment of the diseases/conditions with a combination of pharmaceutically active agents that may be administered separately, the invention further relates to combining separate pharmaceutical compositions in kit form. For example, the kit may comprise two separate pharmaceutical compositions: a compound identified by the present invention; and a second pharmaceutically active compound. The kit comprises a container for containing the separate compositions, such as a divided bottle or a divided foil packet. Additional examples of containers include syringes, boxes, bags, and the like. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of a kit is a blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and a sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen that the tablets or capsules so specified should be ingested. Another example of such a memory aid is a calendar printed on the card, e.g., as follows "First Week, Monday, Tuesday, ...etc.... Second Week, Monday, Tuesday,..." etc. Other variations of memory aids will be readily apparent. A "daily dose" can be a single tablet or capsule or several pills or capsules to be taken on a given day. Also, a daily dose of compound identified by the present invention can consist of one tablet or capsule, while a daily dose of the second compound can consist of several tablets or capsules and vice versa. The memory aid should reflect this and aid in correct administration of the active agents.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter, which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

Methods of formulation are well known in the art and are disclosed, for example, in Remington: The Science and Practice of Pharmacy, Mack Publishing Company, Easton, Pa., 19th Edition (1995). Pharmaceutical compositions for use in the present invention can be in the form of sterile, non-pyrogenic liquid solutions or suspensions, coated capsules, suppositories, lyophilized powders, transdermal patches or other forms known in the art.

The compounds identified by the present invention and other pharmaceutically active compounds, if desired, can be administered to a patient either orally, rectally, parenterally, (for example, intravenously, intramuscularly, or subcutaneously) intracisternally, intravaginally, intraperitoneally, intravesically, locally (for example, powders, ointments or drops), or as a buccal or nasal spray.

Compositions suitable for parenteral injection may comprise physiologically acceptable sterile aqueous or nonaqueous solutions, dispersions, suspensions, or emulsions, and sterile powders for reconstitution into sterile injectable solutions or dispersions. Examples of suitable aqueous and nonaqueous carriers, diluents, solvents, or vehicles include water, ethanol, polyols (propylene glycol, polyethylene glycol, glycerol, and the like), suitable mixtures thereof, triglycerides, including vegetable oils such as olive oil, and injectable organic esters such as ethyl oleate. A preferred carrier is Miglyol®. Proper fluidity can be maintained, for example, by the use of a coating such as lecithin, by the maintenance of the required particle size in the case of dispersions, and by the use of surfactants.

These compositions may also contain adjuvants such as preserving, wetting, emulsifying, and dispersing agents. Prevention of microorganism contamination of the compositions can be ensured by various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents capable of delaying absorption, for example, aluminum monostearate and gelatin.

Solid dosage forms for oral administration include capsules, tablets, powders, and granules. In such solid dosage forms, the active compound is admixed with at least one inert customary excipient (or carrier) such as sodium citrate or dicalcium phosphate or (a) fillers or extenders, as for example, starches, lactose, sucrose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example, paraffin; (f) absorption accelerators, as for example, quaternary ammonium compounds; (g) wetting agents, as for example, cetyl alcohol and glycerol monostearate; (h) adsorbents, as for example, kaolin and bentonite; and/or (i) lubricants, as for example, talc, calcium stearate, magnesium stearate, solid polyethylene glycols, sodium lauryl sulfate, or mixtures thereof. In the case of capsules and tablets, the dosage forms may also comprise buffering agents.

Solid compositions of a similar type may also be used as fillers in soft or hard filled gelatin capsules using such excipients as lactose or milk sugar, as well as high molecular weight polyethylene glycols, and the like.

Solid dosage forms such as tablets, dragees, capsules, and granules can be prepared with coatings and shells, such as enteric coatings and others well known in the art. They may also contain opacifying agents, and can also be of such composition that they release the active compound or compounds in a delayed manner. Examples of embedding compositions that can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil, and sesame seed oil, Miglyol®, glycerol, tetrahydrofurfuryl alcohol, polyethylene glycols and fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

Suspensions, in addition to the active compound, may contain suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Compositions for rectal or vaginal administration are preferably suppositories, which can be prepared by mixing a compound of the present invention with suitable non-irritating excipients or carriers such as cocoa butter, polyethylene glycol or a suppository wax, which are solid at ordinary room temperature, but liquid at body temperature, and therefore, melt in the rectum or vaginal cavity and release the active component.

Dosage forms for topical administration of a compound of the present invention include ointments, powders, sprays and inhalants. The active compound or compounds are admixed under sterile conditions with a physiologically acceptable carrier, and any preservatives, buffers, or propellants that may be required. Opthalmic formulations, eye ointments, powders, and solutions are also contemplated as being within the scope of this invention.

The compounds identified by the present invention may be administered to a patient at dosage levels in the range of about 0.7 to about 7,000 mg per day. For a normal adult human having a body weight of about 70 kg, a dosage in the range of about 0.01 to about 100 mg per kilogram body weight is typically sufficient. The specific dosage and dosage range that can be used depends on a number of factors, including the requirements of the patient, the severity of the condition or disease being treated, and the pharmacological activity of the compound being administered. The determination of dosage ranges and optimal dosages for a particular patient is well within the ordinary skill in of one in the art, particularly in view of this disclosure.

The following paragraphs describe exemplary formulations, dosages etc. useful for non-human animals. The administration of a pharmaceutically active compound can be effected orally or non-orally, for example by injection. An amount of a compound of the present invention is administered such that an effective dose is received, generally a daily dose which, when administered orally to an animal is usually between 0.01 and 1000 mg/kg of body weight, preferably between 0.1 and 50 mg/kg of body weight. Conveniently, the compound can be carried in the drinking water so that a therapeutic dosage of the compound is ingested with the daily water supply. The compound can be directly metered into drinking water, preferably in the form of a liquid, water-soluble concentrate (such as an aqueous solution of a water-soluble salt). Conveniently, the compound can also be added directly to the feed, as such, or in the form of an animal feed supplement, also referred to as a premix or concentrate. A premix or concentrate of the compound in a carrier is more commonly employed for the inclusion of the agent in the feed. Suitable carriers are liquid or solid, as desired, such as water, various meals such as alfalfa meal, soybean meal, cottonseed oil meal, linseed oil meal, corncob meal and corn meal, molasses, urea, bone meal, and mineral mixes such as are commonly employed in poultry feeds. A particularly effective carrier is the respective animal feed itself; that is, a small portion of such feed. The carrier facilitates uniform distribution of the compound in the finished feed with which the premix is blended. It is important that the compound be thoroughly blended into the premix and, subsequently, the feed. In this respect, the compound may be dispersed or dissolved in a suitable oily vehicle such as soybean oil, corn oil, cottonseed oil, and the like, or in a volatile organic solvent and then blended with the carrier. It will be appreciated that the proportions of compound in the concentrate are capable of wide variation since the amount of active compound in the finished feed may be adjusted by blending the appropriate proportion of premix with the feed to obtain a desired level of compound.

High potency concentrates may be blended by the feed manufacturer with proteinaceous carrier such as soybean oil meal and other meals, as described above, to produce concentrated supplements which are suitable for direct feeding to animals. In such instances, the animals are permitted to consume the usual diet. Alternatively, such concentrated supplements may be added directly to the feed to produce a nutritionally balanced, finished feed containing a therapeutically effective level of a compound of the present invention. The mixtures are thoroughly blended by standard procedures, such as in a twin shell blender, to ensure homogeneity.

If the supplement is used as a top dressing for the feed, it likewise helps to ensure uniformity of distribution of the compound across the top of the dressed feed.

Preferred medicated swine, cattle, sheep and goat feed generally contain from 1 to 400 grams of an active compound per ton of feed, the optimum amount for these animals usually being about 50 to 300 grams per ton of feed.

The preferred poultry and domestic pet feeds usually contain about 1 to 400 grams and preferably 10 to 400 grams of an active compound per ton of feed.

For parenteral administration in animals, the compounds of the present invention may be prepared in the form of a paste or a pellet and administered as an implant, usually under the skin of the head or ear of the animal.

In general, parenteral administration involves injection of a sufficient amount of a compound of the present invention to provide the animal with 0.01 to 100 mg/kg of body weight per day of the active ingredient. The preferred dosage for poultry, swine, cattle, sheep, goats and domestic pets is in the range of from 0.1 to 50 mg/kg/day.

Paste formulations can be prepared by dispersing a compound of the present invention in pharmaceutically acceptable oil such as peanut oil, sesame oil, corn oil or the like.

Pellets containing an effective amount of an active compound can be prepared by admixing the compound with a diluent such as carbowax, carnauba wax, and the like, and a lubricant, such as magnesium or calcium stearate, can be added to improve the pelleting process.

It is, of course, recognized that more than one pellet may be administered to an animal to achieve the desired dose level. Moreover, it has been found that implants may also be made periodically during the animal treatment period in order to maintain the proper active agent level in the animal's body.

The methods of treatment of the present invention can also include combination therapy where other pharmaceutically active agents useful for the treatment of female sexual dysfunction are used in combination with the compounds identified by the present invention that attenuate the binding of agouti-related protein to melanocortin receptors, but do not attenuate the binding of α-melanocyte stimulating hormone to melanocortin receptors. For example, compounds that attenuate the binding of agouti-related protein to melanocortin receptors, but do not attenuate the binding of α-melanocyte stimulating hormone to melanocortin receptors can be used in combination with other compounds used to treat female sexual dysfunction.

An "estrogen agonist / antagonist" is a compound that affects some of the same receptors that estrogen does, but not all, and in some instances, it antagonizes or blocks estrogen. It is also known as a "selective estrogen receptor modulator" (SERM). Estrogen agonists / antagonists may also be referred to as antiestrogens although they have some estrogenic activity at some estrogen receptors. Estrogen agonists / antagonists are therefore not what are commonly referred to as "pure antiestrogens". Antiestrogens that can also act as agonists are referred to as Type I antiestrogens. Type I antiestrogens activate the estrogen receptor to bind tightly in the nucleus for a prolonged time but with impaired receptor replenishment (Clark, et al., Steroids 1973;22:707, Capony et al., Mol Cell Endocrinol, 1975;3:233).

An estrogen agonist / antagonist when co-administered with a compound identified by the present invention, either as part of the same pharmaceutical composition or as separate pharmaceutical compositions, is/are effective in treating female sexual dysfunction. By treating female sexual dysfunction, the compositions and methods of the invention are suitable for treating a variety of conditions. These conditions encompass arousal, pain and orgasmic disorders such as: female sexual arousal disorder; hypoactive sexual desire disorder; sexual anhedonia; dyspareunia; and vaginismus. The estrogen agonists / antagonists may be administered systemically or locally. For systemic use, the compounds herein are formulated for parenteral (e.g., intravenous, subcutaneous, intramuscular, intraperitoneal, intranasal or transdermal) or enteral (e.g., oral or rectal) delivery according to conventional methods. Intravenous administration can be by a series of injections or by continuous infusion over an extended period. Administration by injection or other routes of discretely spaced administration can be performed at intervals ranging from weekly to once to three times daily.

Preferred estrogen agonists / antagonists of the present invention include the compounds described in US 5,552,412. Those compounds are described by formula (I) given below: 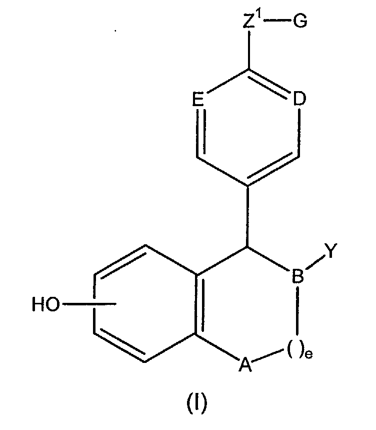 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚ W(CH₂)_{q}-;
   (b) -O(CH₂)ₚ CR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸; 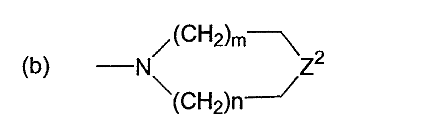 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 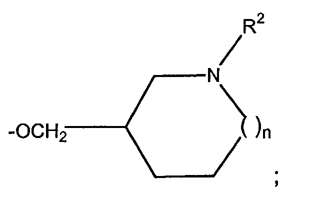
W is
   (a) -CH₂-;
   (b) -CH=CH-;
   (c) -O-;
   (d) -NR²-;
   (e) -S(O)ₙ-;

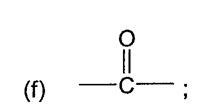

   (g) -CR²(OH)-;
   (h) -CONR²-;
   (i) -NR²CO-;
   (j) 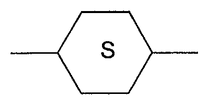 ; or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (I) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
   a ring formed by R⁷ and R⁸ may be optionally fused to a phenyl ring;
   e is 0, 1 or 2;
   m is 1, 2 or 3;
   n is 0, 1 or 2;
   p is 0, 1, 2 or 3;
   q is 0, 1, 2 or 3;
   and optical and geometric isomers thereof; and nontoxic pharmacologically acceptable acid addition salts, N-oxides, esters, quaternary ammonium salts and prodrugs thereof.

By halo is meant chloro, bromo, iodo, or fluoro or by halogen is meant chlorine, bromine, iodine or fluorine.

By alkyl is meant straight chain or branched chain saturated hydrocarbon. Exemplary of such alkyl groups (assuming the designated length encompasses the particular example) are methyl, ethyl, propyl, isopropyl, butyl, sec-butyl, tertiary butyl, pentyl, isopentyl, hexyl and isohexyl.

By alkoxy is meant straight chain or branched chain saturated alkyl bonded through an oxy. Exemplary of such alkoxy groups (assuming the designated length encompasses the particular example) are methoxy, ethoxy, propoxy, isopropoxy, butoxy, isobutoxy, tertiary butoxy, pentoxy, isopentoxy, hexoxy and isohexoxy.

The parenthetical negative or positive sign used herein in the nomenclature denotes the direction plane polarized light is rotated by the particular stereoisomer.

Additional preferred compounds of the invention are disclosed in U.S. Patent No.5,552,412 and are described by formula (IA): 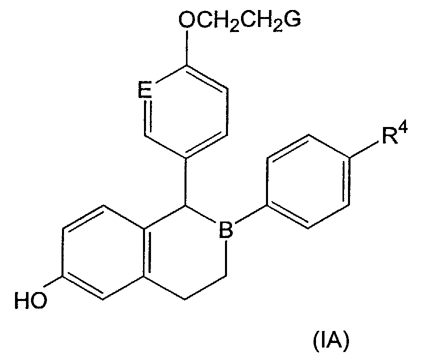 wherein G is 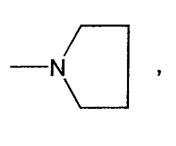 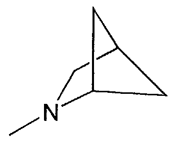 or 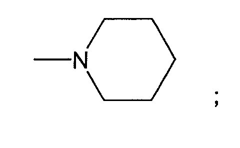 R⁴ is H, OH, F, or Cl; and B and E are independently selected from CH and N.

Especially preferred compounds for the compositions and methods of the invention are:
cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-[4-(2-pyrrolidin-1-yl-ethoxy)-phenyl]-5,6,7,8-tetrahydronaphthalene-2-ol (also known as lasofoxifene);
cis-6-phenyl-5-[4-(2-pyrrolidin-1-yl-ethoxy)-phenyl]-5,6,7,8-tetrahydronaphthalene-2-ol;
cis-1-[6'-pyrrolidinoethoxy-3'-pyridyl]-2-phenyl-6-hydroxy-1,2,3,4-tetrahydronaphthalene;
1-(4'-pyrrolidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol;
1-(4'-pyrrolidinoethoxyphenyl)-2-phenyl-6-hydroxy-1,2,3,4-tetrahydroisoquinoline and pharmaceutically acceptable salts thereof.

An especially preferred salt of (-)-cis-6-phenyl-5-[4-(2-pyrrolidin-1-yl-ethoxy)-phenyl]-5,6,7,8-tetrahydro-naphthalene-2-ol is the tartrate salt.

Other preferred estrogen agonists / antagonists are disclosed in U.S. Patent 5,047,431. The structure of these compounds is given by formula (II) below: 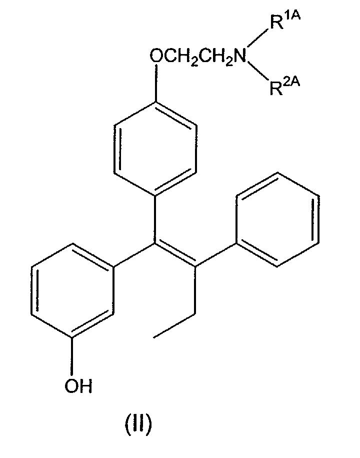 wherein
R^{1A} and R^{2A} may be the same or different and are either H, methyl, ethyl or a benzyl group; and optical or geometric isomers thereof; and pharmaceutically acceptable salts, N-oxides, esters, quaternary ammonium salts, and prodrugs thereof.

Additional preferred estrogen agonists / antagonists are tamoxifen: (ethanamine,2-[-4-(1,2-diphenyl-1-butenyl)phenoxy]-N,N-dimethyl, (Z)-2-, 2-hydroxy-1,2,3-propanetricarboxylate(1:1)) and other compounds as disclosed in U.S. Patent 4,536,516; 4-hydroxy tamoxifen (i.e., tamoxifen wherein the 2-phenyl moiety has a hydroxy group at the 4 position) and other compounds as disclosed in U.S. Patent 4,623,660; droloxifene: (3-hydroxytamoxifen; or (*E*)-3-[1-[4-[2-(dimethyamino)ethoxy phenyl-1-butenyl]phenol); raloxifene: (methanone, [6-hydroxy-2-(4-hydroxyphenyl) benzo[b]thien-3-yl][4-[2-(1-piperidinyl)ethoxy]phenyl]-,hydrochloride) and other compounds as disclosed in U.S. Patents 4,418,068; 5,393,763; 5,457,117; 5,478,847 and 5,641,790; toremifene: (ethanamine, 2-[4-(4-chloro-1,2-diphenyl-1-butenyl)phenoxy]-N,N-dimethyl-, (Z)-, 2-hydroxy-1,2,3-propanetricarboxylate (1:1) and other compounds as disclosed in U.S. Patents 4,696,949 and 4,996,225; centchroman: 1-[2-[[4-(-methoxy-2,2, dimethyl-3-phenyl-chroman-4-yl)-phenoxy]-ethyl]-pyrrolidine and other compounds as disclosed in U.S. Patent 3,822,287; idoxifene: pyrrolidine, 1-[-[4-[[1-(4-iodophenyl)-2-phenyl-1-butenyl]phenoxy]ethyl] and other compounds as disclosed in U.S. Patent 4,839,155; 6-(4-hydroxy-phenyl)-5-[4-(2-piperidin-1-yl-ethoxy)-benzyl]-naphthalen -2-ol and other compounds as disclosed in U.S. Patent 5,484,795; and {4-[2-(2-aza-bicyclo[2.2.1]hept-2-yl)-ethoxy]-phenyl}-[6-hydroxy-2-(4-hydroxy-phenyl)-benzo[b]thiophen-3-yl]-methanone and other compounds as disclosed in published international patent application WO 95/10513. Other preferred compounds include GW 5638 and GW 7604, the synthesis of which is described in Willson et al., J. Med. Chem., 1994: 37: 1550-1552.

Further preferred estrogen agonists / antagonists include EM-652 (as shown in formula (III) and EM-800 (as shown in formula (IV)). The synthesis of EM-652 and EM-800 and the activity of various enantiomers is described in Gauthier et al., J. Med. Chem., 1997;40:2117-2122.

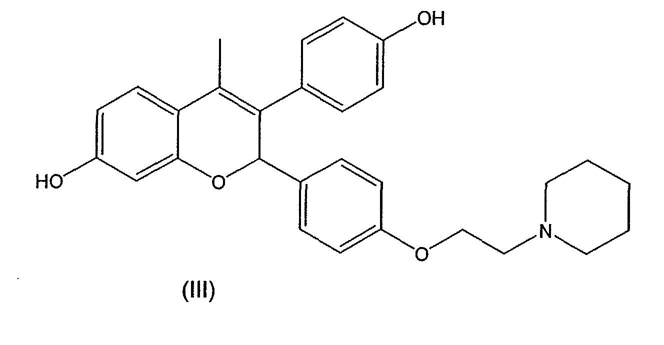

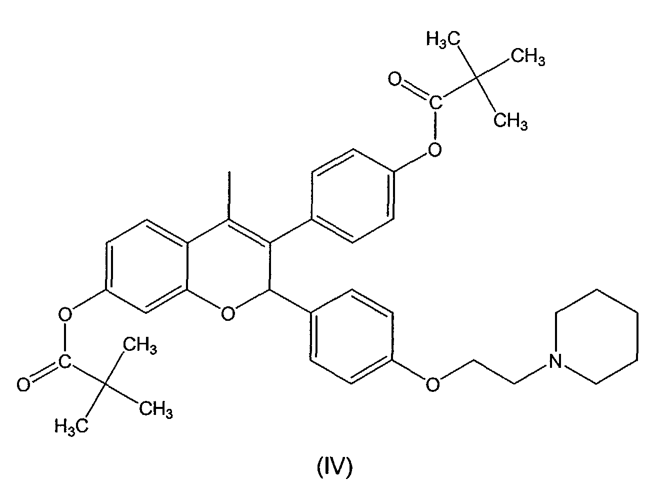

Further preferred estrogen agonists / antagonists include TSE-424 and other compounds disclosed in U.S. Patent 5,998,402, U.S. Patent 5,985,910, U.S. Patent 5,780,497, U.S. Patent 5,880,137, and European Patent Application EP 0802183 A1 including the compounds of the formulas V and VI, below: 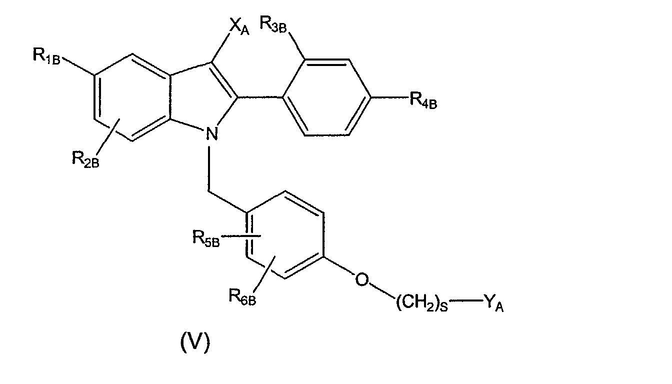 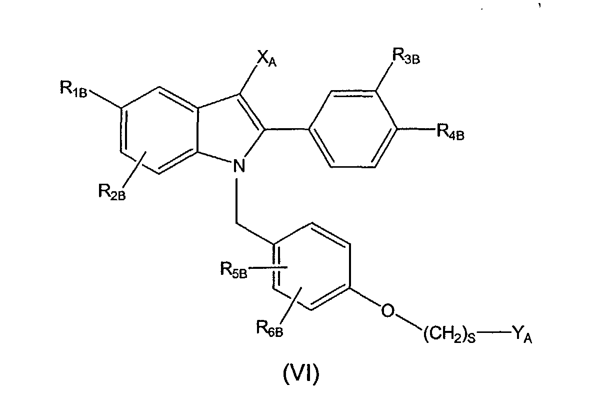 wherein:
R_{1B} is selected from H, OH or the C₁-C₁₂ esters (straight chain or branched) or C₁-C₁₂ (straight chain or branched or cyclic) alkyl ethers thereof, or halogens; or C₁-C₄ halogenated ethers including triflouromethyl ether and trichloromethyl ether.
R_{2B}, R_{3B}, R_{4B}, R_{5B}, and R_{6B} are independently selected from H, OH or the C₁-C₁₂ esters (straight chain or branched) or C₁-C₁₂ alkyl ethers (straight chain or branched or cyclic) thereof, halogens, or C₁-C₄ halogenated ethers including triflouromethyl ether and trichloromethyl ether, cyano, C₁-C₆ alkyl (straight chain or branched), or trifluoromethyl, with the proviso that, when R_{1B} is H, R_{2B} is not OH.
X_{A} is selected from H, C₁-C₆ alkyl, cyano, nitro, triflouromethyl, and halogen;
s is 2 or 3;
Y_{A} is selected from:
a) the moiety: 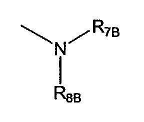 wherein R_{7B} and R_{8B} are independently selected from the group of H, C₁-C₆ alkyl, or phenyl optionally substituted by CN, C₁-C₆ alkyl (straight chain or branched), C₁-C₆ alkoxy (straight chain or branched), halogen, -OH, -CF₃, or -OCF₃;
b) a five-membered saturated, unsaturated or partially unsaturated heterocycle containing up to two heteroatoms selected from the group consisting of - O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
c) a six-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
d) a seven-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl; or
e) a bicyclic heterocycle containing from 6-12 carbon atoms either bridged or fused and containing up to two heteroatoms selected from the group consisting of -O-, -NH-, -N(C₁-C₄ alkyl)-, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -N=, C₁-C₄ alkylamino, di(C₁-C₄)alkylamino,-NHSO₂R_{1B}, -NHCOR_{1B}, -NO₂, and phenyl optionally substituted with 1-3 (C₁-C₄) alkyl; and optical or geometric isomers thereof; and nontoxic pharmacologically acceptable acid addition salts, N-oxides, esters, quaternary ammonium salts, and prodrugs thereof.

The more preferred compounds of this invention are those having the general structures V or VI, above, wherein:
R_{1B} is selected from H, OH or the C₁-C₁₂ esters or alkyl ethers thereof, and halogen;
R_{2B}, R_{3B}, R_{4B}, R_{5B}, and R_{6B} are independently selected from H, OH or the C₁-C₁₂ esters or alkyl ethers thereof, halogen, cyano, C₁-C₆ alkyl, or trihalomethyl, preferably trifluoromethyl, with the proviso that, when R_{1B} is H, R_{2B} is not OH;
X_{A} is selected from H, C₁-C₆ alkyl, cyano, nitro, triflouromethyl, and halogen;
Y_{A} is the moiety: 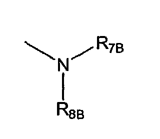
R_{7B} and R_{8B} are selected independently from H, C₁-C₆ alkyl, or combined by -(CH₂)_{w}-, wherein w is an integer of from 2 to 6, so as to form a ring, the ring being optionally substituted by up to three substituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄), -NH₂, C₁-C₄ alkylamino, C₁-C₄ dialkylamino, -NHSO₂(C₁-C₄), -HNCO(C₁-C₄), and -NO₂; and optical and geometric isomers thereof; and nontoxic pharmacologically acceptable acid addition salts, N-oxides, esters, quaternary ammonium salts, and prodrugs thereof.

The rings formed by a concatenated R_{7B} and R_{8B}, mentioned above, may include, but are not limited to, aziridine, azetidine, pyrrolidine, piperidine, hexamethyleneamine or heptamethyleneamine rings.

The most preferred compounds of structural formulas V and VI, above, are those wherein R_{1B} is OH; R_{2B} - R_{6B} are as defined above; X_{A} is selected from the group of Cl, NO₂, CN, CF₃, or CH₃; Y_{A} is the moiety 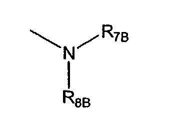 and R_{7B} and R_{8B} are concatenated together as -(CH₂)ₜ-, wherein t is an integer of from 4 to 6, to form a ring optionally substituted by up to three subsituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄)alkyl, -NH₂, C₁-C₄ alkylamino, di(C₁-C₄)alkylamino, -NHSO₂(C₁-C₄)alkyl, -NHCO(C₁-C₄)alkyl, and -NO₂; and optical and geometric isomers thereof; and nontoxic pharmacologically acceptable acid addition salts, N-oxides, esters, quaternary ammonium salts, and prodrugs thereof including the compound, TSE-424, of formula (Va) below:

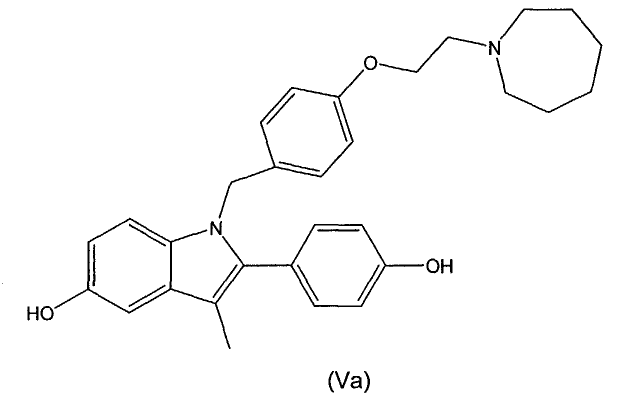

The pharmaceutically acceptable acid addition salts of the estrogen agonists / antagonists of this invention may be formed of the compound itself, or of any of its esters, and include the pharmaceutically acceptable salts which are often used in pharmaceutical chemistry. For example, salts may be formed with inorganic or organic acids such as hydrochloric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like, most preferable with hydrochloric acid, citric acid, benzoic acid, maleic acid, acetic acid and propionic acid.

The estrogen agonists / antagonists of this invention, as discussed above, can be administered in the form of acid addition salts. The salts are conveniently formed by reacting a compound, if basic, with a suitable acid, such as have been described above. The salts are quickly formed in high yields at moderate temperatures, and often are prepared by merely isolating the compound from a suitable acidic wash as the final step of the synthesis. The salt-forming acid is dissolved in an appropriate organic solvent, or aqueous organic solvent, such as an alkanol, ketone or ester. On the other hand, if the compound of this invention is desired in the free base form, it is isolated from a basic final wash step, according to the usual practice. A preferred technique for preparing hydrochlorides is to dissolve the free base in a suitable solvent and dry the solution thoroughly, as over molecular sieves, before bubbling hydrogen chloride gas through it. A preferred salt of (-)-cis-6-phenyl-5-[4-(2-pyrrolidin-1-yl-ethoxy)-phenyl]-5,6,7,8-tetrahydronaphthalene-2-ol is the D-(-)-tartrate salt. This compound may also be named as follows: 2-naphthalenol 5,6,7,8-tetrahydro-6-phenyl-5[4-[2-(1-pyrrolidinyl)ethoxy]phenyl],(5R-cis)],[S-(R*, R*)]-2,3-dihydroxybutanedionate (1:1). It will also be recognized that it is possible to administer amorphous forms of the estrogen agonists / antagonists.

For the treatment of female sexual dysfunction, a cGMP elevator agent may be coadministered with a compound identified by the present invention either separately or in a single composition.

Preferred as the cGMP elevator are cGMP PDE inhibitors. cGMP PDE inhibitors which are selective for cGMP PDEs rather than cyclic adenosine 3',5'-monophosphate phosphodiesterases (cAMP PDEs) and/or which are selective inhibitors of the cGMP PDE_{V} isoenzyme are particularly preferred. Such particularly preferred cGMP PDE inhibitors are disclosed in US patents 5,250,534; 5,346,901; 5,272,147, and in the international patent application published as WO 94/28902 designating, *inter alia,* the U.S., each of which is incorporated herein by reference.

Preferred cGMP PDE_{V} (also called PDE5) inhibitors include compounds of formula (VII): 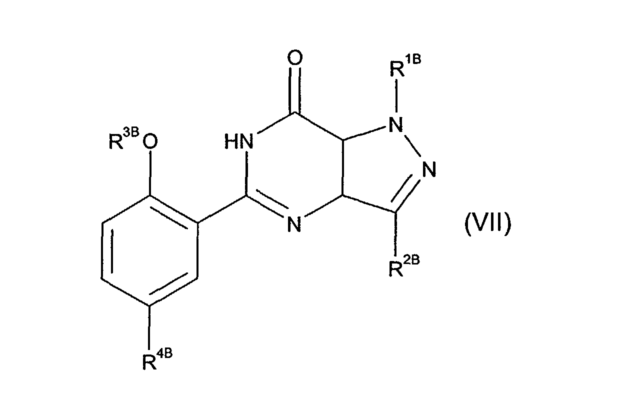 wherein:
R^{1B} is H; C₁-C₃ alkyl; C₁-C₃ perfluoroalkyl; or C₃-C₅ cycloalkyl;
R^{2B} is H; C₁-C₆ alkyl optionally substituted with C₃-C₆ cycloalkyl; C₁-C₃ perfluoroalkyl; or C₃-C₆ cycloalkyl;
R^{3B} is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R^{4B} is C₁-C₄ alkyl optionally substituted with OH, NR^{5B}R^{6B}, CN, CONR^{5B}R^{6B} or CO₂R^{7B}; C₂-C₄ alkenyl optionally substituted with CN, CONR^{5B}R^{6B} or CO₂R^{7B}; C₂-C₄ alkanoyl optionally substituted with NR^{5B}R^{6B}; (hydroxy)C₂-C₄ alkyl optionally substituted with NR^{5B}R^{6B}; (C₂-C₃ alkoxy)C₁-C₂ alkyl optionally substituted with OH or NR^{5B}R^{6B}; CONR^{5B}R⁶B CO₂R^{7B}; halo; NR^{5B}R^{6B}; NHSO₂NR^{5B}R^{6B}; NHSO₂R^{8B}; SO₂NR^{9B}R^{10B} or phenyl, pyridyl, pyrimidinyl, imidazolyl, oxazolyl, thiazolyl, thienyl or triazolyl any of which is optionally substituted with methyl;
R^{5B} and R^{6B} are each independently H or C₁-C₄ alkyl, or together with the nitrogen atom to which they are attached form a pyrrolidinyl, piperidino, morpholino, 4-N(R^{11B})-piperazinyl or imidazolyl group wherein said group is optionally substituted with methyl or OH;
R^{7B} is H or C₁-C₄ alkyl;
R^{8B} is C₁-C₃ alkyl optionally substituted with NR^{5B}R^{6B};
R^{9B} and R^{10B} together with the nitrogen atom to which they are attached form a pyrrolidinyl, piperidino, morpholino or 4-N(R^{12B})-piperazinyl group wherein said group is optionally substituted with C₁-C₄ alkyl, C₁-C₃ alkoxy, NR^{13B}R^{14B} or CONR^{13B}R^{14B};
R^{11B} is H; C₁-C₃ alkyl optionally substituted with phenyl; (hydroxy)C₂-C₃ alkyl; or C₁-C₄ alkanoyl;
R^{12B} is H; C₁-C₆ alkyl; (C₁-C₃ alkoxy)C₂-C₆ alkyl; (hydroxy)C₂-C₆ alkyl; (R^{13B}R^{14B}N)C₂-C₆ alkyl; (R^{13B}R^{14B}NOC)C₁-C₆ alkyl; CONR^{13B}R^{14B}; CSNR^{13B}R^{14B}; or C(NH)NR^{13B}R^{14B}; and
R^{13B} and R^{14B} are each independently H; C₁-C₄ alkyl; (C₁-C₃ alkoxy)C₂-C₄ alkyl; or (hydroxy)C₂-C₄ alkyl;
or a pharmaceutically acceptable salt thereof;
or a pharmaceutically acceptable composition containing either entity.

Preferred cGMP PDE_{V} inhibitors include sildenafil (preferably the citrate salt) (Viagra®) {1-[[3-(6,7-dihydro-1-methyl-7-oxo-3-propyl-1H-pyrazolo[4,3-d]pyrimidin-5-yl)-4-ethoxy-phenyl]sulfonyl]-4-methylpiperazine}, which has the structure of formula (VIII): 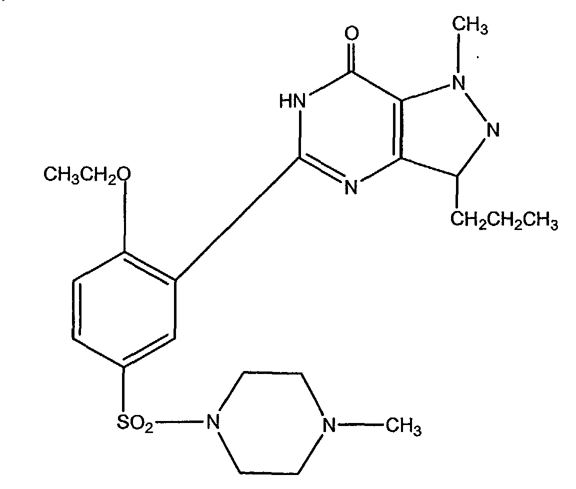 and pharmaceutically acceptable salts thereof, the compound having the structure of formula (IX): 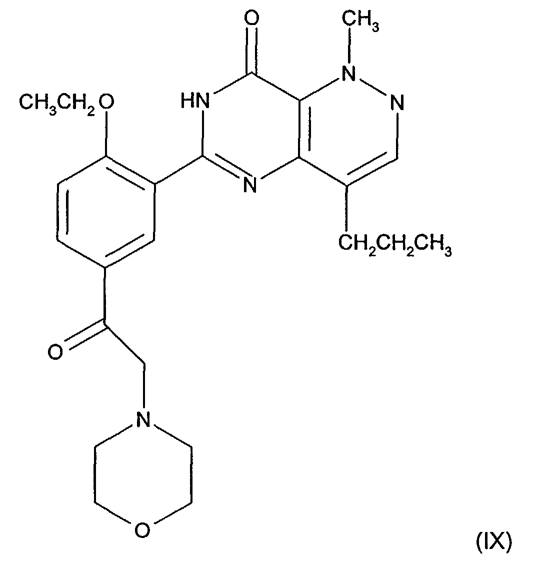 and pharmaceutically acceptable salts thereof, and the compound, 3-ethyl-5-{5-[(4-ethylpiperazino) sulphonyl]-2-(2-methoxyethoxy)pyrid-3-yl}-2-(2-pyridylmethyl)-6,7-dihydro-2H-pyrazolo[4,3-d]pyrimidin-7-one of formula (X) below:

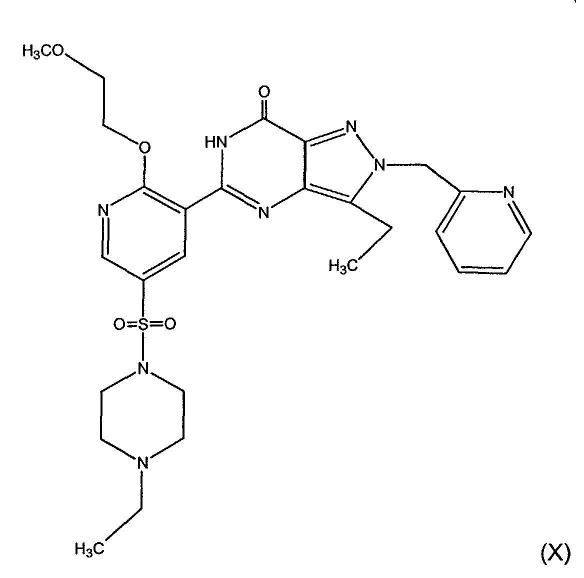

The compound of formula (IX) is disclosed, for example, in US Patents 5,272,147 and 5,426,107.

Also preferred as cGMP PDE_{V} inhibitors are compounds disclosed in PCT/EP95/00183, published as WO 95/19978 and which designates, *inter alia,* the United States, herein incorporated by reference, said compounds having the formula (XI): 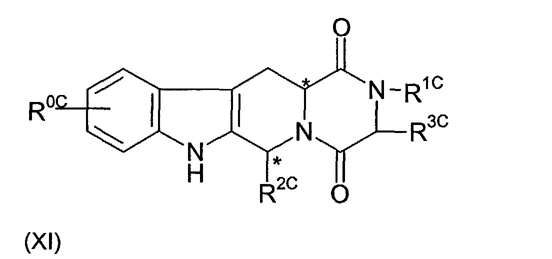 and salts and solvates thereof, in which:
R^{0C} represents hydrogen, halogen or C₁-C₆alkyl,;
R^{1C} represents hydrogen, C₁-C₆alkyl, C₂-C₆alkenyl, C₂-C₆alkynyl, haloC₁-C₆alkyl, C₃-C₈cycloalkyl, C₃-C₈cycloalkylC₁-C₃alkyl, arylC₁-C₃alkyl or heteroarylC₁-C₃alkyl;
R^{2C} represents an optionally substituted monocyclic aromatic ring selected from benzene, thiophene, furan and pyridine or an optionally substituted bicyclic ring 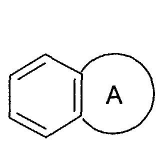 attached to the rest of the molecule via one of the benzene ring carbon atoms and wherein the fused ring A is 5- or 6- membered ring which may be saturated or partially or fully unsaturated and comprises carbon atoms and optionally one or two heteroatoms selected from oxygen, sulphur and nitrogen; and R^{3C} represents hydrogen or C₁-C₃alkyl, or R^{1C} and R^{3C} together represent a 3- or 4- membered alkyl or alkenyl ring.

A preferred subset of compound having formula XIa (also disclosed in WO 95/19978) includes compounds of the formula: 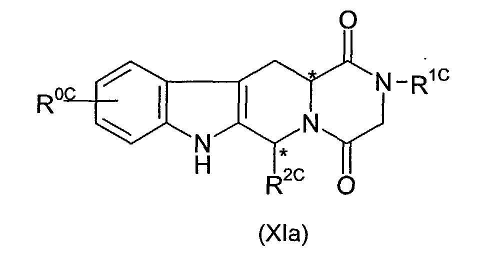 and salts and solvates thereof, in which:
R^{0C} represents hydrogen, halogen or C₁-C₆alkyl;
R^{1C} represents hydrogen, C₁-C₆alkyl, haloC₁-C₆alkyl, C₃-C₈cycloalkyl,
C₃-C₈cycloalkyl-C₁-C₃alkyl, arylC₁-C₃alkyl or heteroarylC₁-C₃alkyl; and
R^{2C} represents an optionally substituted monocyclic aromatic ring selected from benzene, thiophene, furan and pyridine or an optionally substituted bicyclic ring 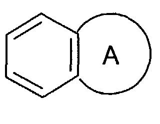 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen.

Suitable cGMP PDE5 inhibitors for the use according to the present invention include: the pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed in EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the compounds disclosed in published international application WO95/19978; the compounds disclosed in published international application WO 99/24433 and the compounds disclosed in published international application WO 93/07124.

It is to be understood that the contents of the above published patent applications, and in particular the general formulae and exemplified compounds therein are incorporated herein in their entirety by reference thereto.

Preferred type V phosphodiesterase inhibitors for the use according to the present invention include: 5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) also known as 1-[[3-(6,7-dihydro-1-methyl-7-oxo-3-propyl-1 H-pyrazolo[4,3-d]pyrimidin-5-yl)-4-ethoxyphenyl]sulphonyl]-4-methylpiperazine (see EP-A-0463756); 5-(2-ethoxy-5-morpholinoacetylphenyl)-1-methyl-3-n-propyl-1,6-dihydro-7H-pyrazolo[4,3-d]pyrimidin-7-one (see EP-A-0526004);
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (see WO98/49166); 3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (see WO99/54333);
(+)-3-ethyl-5-[5-(4-ethylpiperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazolo[4,3-d] pyrimidin-7-one (see WO99/54333);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see Example 1 hereinafter); 5-[2*-iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see Example 2 hereinafter);
5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see Example 3 hereinafter); 5-(5-Acetyl-2-propoxy-3-pyridinyl)-3-ethyl-2-(1-isopropyl-3-azetidinyl)-2,6-dihydro-7*H*-pyrazolo[4,3-*d*]pyrimidin-7-one (see Example 4 hereinafter); 5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one (see Example 5 hereinafter); (6R,12aR)-2,3,6,7,12,12a-hexahydro-2-methyl-6-(3,4-methylenedioxyphenyl) - pyrazino[2',1':6,1]pyrido[3,4-b]indole-1,4-dione (IC-351) (Cialis®), i.e. the compound of examples 78 and 95 of published international application WO95/19978, as well as the compound of examples 1, 3, 7 and 8;
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) (BAY38-9456) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e., the compound of examples 20, 19, 337 and 336 of published international application WO99/24433; and the compound of example 11 of published international application WO93/07124 (EISAI); and compounds 3 and 14 from Rotella D P, J. Med. Chem., 2000, 43, 1257.

Still other type cGMP PDE5 inhibitors useful in conjunction with the present invention include:4-bromo-5-(pyridylmethylamino)-6-[3-(4-chlorophenyl)-propoxy]-3(2H)pyridazinone; 1-[4-[(1,3-benzodioxol-5- ylmethyl)amiono]-6-chloro-2-quinozolinyl]-4-piperidine-carboxylic acid, monosodium salt; (+)-cis-5,6a,7,9,9,9a-hexahydro-2-[4-(trifluoromethyl)-phenylmethyl-5-methyl-cyclopent-4,5]imidazo[2,1-b]purin-4(3H)one; furazlocillin; cis-2-hexyl-5-methyl-3,4,5,6a,7,8,9,9a-octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3-(2H)pyridazinone; I-methyl-5(5-morpholinoacetyl-2-n-propoxyphenyl)-3-n-propyl-1,6-dihydro- 7H-pyrazolo(4,3-d)pyrimidin-7-one; 1-[4-[(1,3-benzodioxol-5-ylmethyl)arnino]-6-chloro-2- quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisai); Bay-38-3045 & 38-9456 (Bayer) and Sch-51866.

The suitability of any particular cGMP PDE5 inhibitor can be readily determined by evaluation of its potency and selectivity using literature methods followed by evaluation of its toxicity, absorption, metabolism, pharmacokinetics, etc in accordance with standard pharmaceutical practice.

Preferably, the cGMP PDE5 inhibitors have an IC₅₀ at less than 100 nanomolar, more preferably, at less than 50 nanomolar, more preferably still at less than 10 nanomolar.

IC₅₀ values for the cGMP PDE5 inhibitors may be determined using established literature methodology, for example as described in EP0463756-B1 and EP0526004-A1.

Preferably the cGMP PDE5 inhibitors used are selective for the PDE5 enzyme. Preferably they are selective over PDE3, more preferably over PDE3 and PDE4. Preferably, the cGMP PDE5 inhibitors have a selectivity ratio greater than 100 more preferably greater than 300, over PDE3 and more preferably over PDE3 and PDE4.

Selectivity ratios may readily be determined by the skilled person. IC50 values for the PDE3 and PDE4 enzyme may be determined using established literature methodology, see S A Ballard *et al*., Journal of Urology, 1998, vol. 159, pages 2164-2171.

### cGMP Example 1

2-(Methoxyethyl)-5-[2-ethoxy-5-(4-ethylpiperazin-1-ylsulphonyl)pyridin-3-yl]-3-ethyl-2,6-dihydro-7H-pyrazolo[4,3-d]pyrimidin-7-one 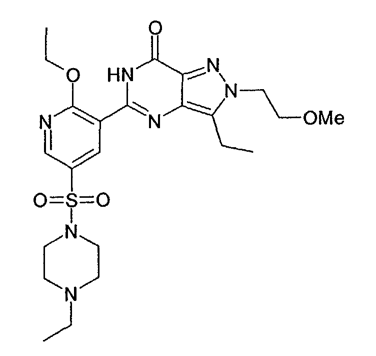 A mixture of the product from stage i) below (0.75mmol), potassium bis(trimethylsilyl)amide (298mg, 1.50mmol) and ethyl acetate (73 microlitres, 0.75mmol) in ethanol (10ml) was heated at 120°C in a sealed vessel for 12 hours. The cooled mixture was partitioned between ethyl acetate and aqueous sodium bicarbonate solution, and the layers separated. The organic phase was dried (MgSO₄), and evaporated under reduced pressure. The crude product was purified by column chromatography on silica gel using dichloromethane:methanol (98:2) as eluant to afford the title compound, 164mg; Found : C, 53.18; H, 6.48; N, 18.14; C₂₃H₃₃N₇O₅S;0.20C₂H₅CO₂CH₃ requires C, 53.21; H, 6.49; N, 18.25%; δ (CDCl₃): 1.04 (3H, t), 1.40 (3H, t), 1.58 (3H, t), 2.41 (2H, q), 2.57 (4H, m), 3.08 (2H, q), 3.14 (4H, m), 3.30 (3H, s), 3.92 (2H, t), 4.46 (2H, t), 4.75 (2H, q), 8.62 (1H, d), 9.04 (1H, d), 10.61 (1H, s); LRMS : m/z 520 (M+1)⁺; mp 161-162°C.

### Preparation of Starting Materials

### a) Pyridine-2-amino-5-sulphonic acid

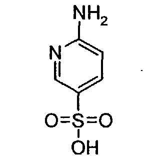

2-Aminopyridine (80g, 0.85mol) was added portionwise over 30 minutes to oleum (320g) and the resulting solution heated at 140°C for 4 hours. On cooling, the reaction was poured onto ice (200g) and the mixture stirred in an ice/salt bath for a further 2 hours. The resulting suspension was filtered, the solid washed with ice water (200ml) and cold IMS (200ml) and dried under suction to afford the title compound as a solid, 111.3g; LRMS : m/z 175 (M+1)⁺

### b) Pyridine-2-amino-3-bromo-5-sulphonic acid

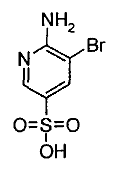

Bromine (99g, 0.62mol) was added dropwise over an hour, to a hot solution of the product from stage a) (108g, 0.62mol) in water (600ml) so as to maintain a steady reflux. Once the addition was complete the reaction was cooled and the resulting mixture filtered. The solid was washed with water and dried under suction to afford the title compound, 53.4g; δ (DMSOd₆, 300MHz): 8.08 (1H, s), 8.14 (1H, s); LRMS : m/z 253 (M)⁺.

### c) Pvridine-3-bromo-2-chloro-5-sulphonyl chloride

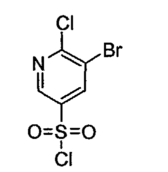

A solution of sodium nitrite (7.6g, 110.0mmol) in water (30ml) was added dropwise to an ice-cooled solution of the product from stage b) (25.3g, 100.0mmol) in aqueous hydrochloric acid (115ml, 20%), so as to maintain the temperature below 6°C. The reaction was stirred for 30 minutes at 0°C and for a further hour at room temperature. The reaction mixture was evaporated under reduced pressure and the residue dried under vacuum at 70°C for 72 hours. A mixture of this solid, phosphorus pentachloride (30.0g, 144mmol) and phosphorus oxychloride (1ml, 10.8mmol) was heated at 125°C for 3 hours, and then cooled. The reaction mixture was poured onto ice (100g) and the resulting solid filtered, and washed with water. The product was dissolved in dichloromethane, dried (MgSO₄), and evaporated under reduced pressure to afford the title compound as a yellow solid, 26.58g; δ (CDCl_{3,} 300MHz): 8.46 (1H, s), 8.92 (1H, s).

### d) 3-Bromo-2-chloro-5-(4-ethylpiperazin-1-ylsulphonyl)pyridine

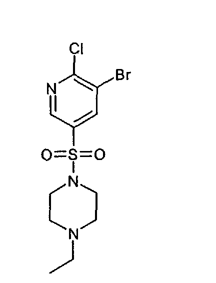

A solution of 1-ethylpiperazine (11.3ml, 89.0mmol) and triethylamine (12.5ml, 89.0mmol) in dichloromethane (150ml) was added dropwise to an ice-cooled solution of the product from stage c) (23.0g, 79.0mmol) in dichloromethane (150ml) and the reaction stirred at 0°C for an hour. The reaction mixture was concentrated under reduced pressure and the residual brown oil was purified by column chromatography on silica gel, using an elution gradient of dichloromethane:methanol (99:1 to 97:3) to afford the title compound as an orange solid, 14.5g; δ (CDCl₃, 300MHz): 1.05 (3H, t), 2.42 (2H, q), 2.55 (4H, m), 3.12 (4H, m), 8.24 (1H, s), 8.67 (1H, s).

### e) 3-Bromo-2-ethoxy-5-(4-ethylpiperazin-1-ylsulphonyl)pyridine

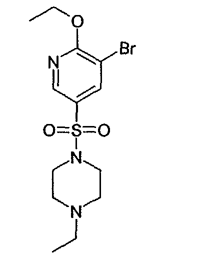

A mixture of the product from stage d) (6.60g, 17.9mmol) and sodium ethoxide (6.09g, 89.55mmol) in ethanol (100ml) was heated under reflux for 18 hours, then cooled. The reaction mixture was concentrated under reduced pressure, the residue partitioned between water (100ml) and ethyl acetate (100ml), and the layers separated. The aqueous phase was extracted with ethyl acetate (2x100ml), the combined organic solutions dried (MgSO₄) and evaporated under reduced pressure to afford the title compound as a brown solid, 6.41g; Found : C, 41.27; H, 5.33; N, 11.11. C₁₃H₂₀BrN₃O₃S requires C, 41.35; H, 5.28; N, 10.99%; δ (CDCl_{3,} 300MHz): 1.06 (3H, t), 1.48 (3H, t), 2.42 (2H, q), 2.56 (4H, m), 3.09 (4H, m), 4.54 (2H, q), 8.10 (1H, s), 8.46 (1H, s); LRMS : m/z 378, 380 (M+1)⁺.

### f) Pyridine 2-ethoxy-5-(4-ethylpiperazin-1-ylsulphonyl)-3-carboxylic acid ethyl ester

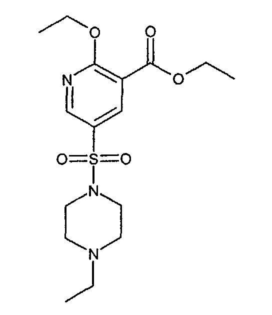

A mixture of the product from stage e) (6.40g, 16.92mmol), triethylamine (12ml, 86.1mmol), and palladium (0) tris(triphenylphosphine) in ethanol (60ml) was heated at 100°C and 200 psi, under a carbon monoxide atmosphere, for 18 hours, then cooled. The reaction mixture was evaporated under reduced pressure and the residue purified by column chromatography on silica gel, using an elution gradient of dichloromethane:methanol (100:0 to 97:3) to afford the title compound as an orange oil, 6.2g; δ (CDCl_{3,} 300MHz): 1.02 (3H, t), 1.39 (3H, t), 1.45 (3H, t), 2.40 (2H, q), 2.54 (4H, m), 3.08 (4H, m), 4.38 (2H, q), 4.55 (2H, q), 8.37 (1H, s), 8.62 (1H, s); LRMS: m/z 372 (M+1)⁺

### g) Pyridine 2-ethoxy-5-(4-ethylpiperazin-1-ylsulphonyl)-3-carboxylic acid

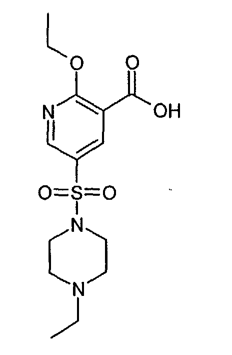

A mixture of the product from stage f) (4.96g, 13.35mmol) and aqueous sodium hydroxide solution (25ml, 2N, 50.0mmol) in ethanol (25ml) was stirred at room temperature for 2 hours. The reaction mixture was concentrated under reduced pressure to half it's volume, washed with ether and acidified to pH 5 using 4N hydrochloric acid. The aqueous solution was extracted with dichloromethane (3x30ml), the combined organic extracts dried (MgSO₄) and evaporated under reduced pressure to afford the title compound as a tan coloured solid, 4.02g; δ (DMSOd₆, 300MHz): 1.18 (3H, t), 1.37 (3H, t), 3.08 (2H, q), 3.17-3.35 (8H, m), 4.52 (2H, q), 8.30 (1H, s), 8.70 (1H, s).

### h) 4-[2-Ethoxy-5-(4-ethylpiperazin-1-ylsulphonyl)pyridin-3-ylcarboxamido]-1H-3-ethylpyrazole-5-carboxamide

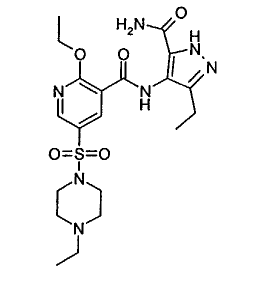

A solution of 4-amino-3-ethyl-1H-pyrazole-5-carboxamide (WO 9849166, preparation 8) (9.2g, 59.8mmol) in N,N-dimethylformamide (60ml) was added to a solution of the product from stage g) (21.7g, 62.9mmol), 1-hydroxybenzotriazole hydrate (10.1g, 66.0mmol) and triethylamine (13.15ml, 94.3mmol) in dichloromethane (240ml). 1-(3-Dimethylaminopropyl)-3-ethylcarbodiimide hydrochloride (13.26g, 69.2mmol) was added and the reaction stirred at room temperature for 6 hours. The dichloromethane was removed under reduced pressure, the remaining solution poured into ethyl acetate (400ml), and this mixture washed with aqueous sodium bicarbonate solution (400ml). The resulting crystalline precipitate was filtered, washed with ethyl acetate and dried under vacuum, to afford the title compound, as a white powder, 22g; δ (CDCl₃+1 drop DMSOd₆) 0.96 (3H, t), 1.18 (3H, t), 1.50 (3H, t), 2.25-2.56 (6H, m), 2.84 (2H, q), 3.00 (4H, m), 4.70 (2H, q), 5.60 (1H, br s), 6.78 (1H, br s), 8.56 (1H, d), 8.76 (1H, d), 10.59 (1H, s), 12.10-12.30 (1H, s); LRMS: m/z 480 (M+1)⁺.

### i) 2-Methoxyethyl-4-[2-ethoxy-5-(4-ethylpiperazin-1-ylsulphonyl)pyridin-3-vlcarboxamidol-3-ethvlpyrazole-5-carboxamide

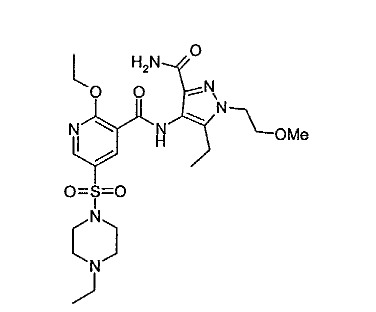

1-Bromo-2-methoxyethane (1.72mmol) was added to a solution of the product from stage h) (750mg, 1.56mmol) and caesium carbonate (1.12g, 3.44mmol) in N,N-dimethylformamide (15ml) and the reaction stirred at 60°C for 18 hours. The cooled mixture was partitioned between water and ethyl acetate, and the layers separated. The organic layer was dried (MgSO₄), concentrated under reduced pressure and azeotroped with toluene to give a solid. This product was recrystallised from ether, to afford the title compound as a white solid.

### cGMP Example 2

### 5-r2-iso-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one

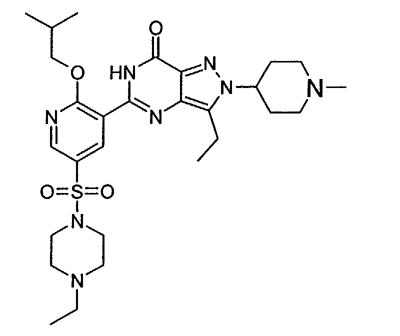

A mixture of the product from stage b) below (90mg, 0.156mmol), potassium bis(trimethylsilyl)amide (156mg, 0.78mmol) and ethyl acetate (14mg, 0.156mmol) in iso-propanol (12ml) was stirred at 130°C for 6 hours in a sealed vessel. The cooled reaction mixture was poured into saturated aqueous sodium bicarbonate solution (60ml), and extracted with ethyl acetate (60ml). The combined organic extracts were dried (MgSO₄), and evaporated under reduced pressure to give a gum. The crude product was purified by column chromatography on silica gel using dichloromethane:methanol:0.88 ammonia (92.6:6.6:0.6) to afford the title compound as a beige foam, 36 mg; δ (CDCl₃) 1.01 (3H, t), 1.12 (6H, d), 1.39 (3H, t), 1.94 (2H, m), 2.15 (2H, m), 2.22-2.44 (6H, m), 2.55 (6H, m), 3.02 (4H, m), 3.14 (4H, m), 4.22 (1H, m), 4.43 (2H, d), 8.60 (1H, d), 9.00 (1H, d), 10.54 (1H, s).

### Preparation of Starting Materials

### a) 2-(1-tert-Butoxycarbonylpiperidin-4-yl)-4-[2-ethoxy-5-(4-ethylpiperazin-1-ylsulphonyl)pyridin-3-ylcarboxamido]-3-ethylpyrazole-5-carboxamide

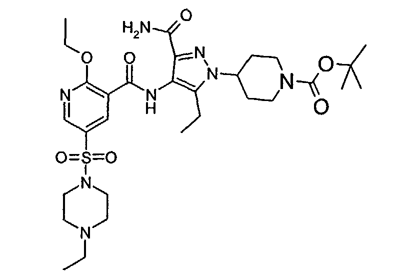

Sodium hydride (64mg, 60% dispersion in mineral oil, 1.6mmol) was added to a solution of the product from Example 1, stage h) (1.46mmol) in tetrahydrofuran (10ml), and the solution stirred for 10 minutes. *tert*-Butyl 4-[(methylsulphonyl)oxy]-1-piperidinecarboxylate (WO 9319059) (1.60mmol) was added and the reaction stirred at 60°C for 3 days. The cooled mixture was partitioned between ethyl acetate and aqueous sodium bicarbonate solution, and the phases separated. The aqueous layer was extracted with ethyl acetate, the combined organic solutions dried (MgSO₄) and evaporated under reduced pressure. The residue was purified by column chromatography on silica gel using dichloromethane:methanol (98:2) as eluant to afford the title compound as a white foam, 310 mg; δ (CDCl₃) 1.02 (3H, t), 1.23 (3H, t), 1.49 (9H, s), 1.57 (3H, m), 1.93 (2H, m), 2.16 (2H, m), 2.40 (2H, q), 2.54 (4H, m), 2.82-2.97 (4H, m), 3.10 (4H, m), 4.30 (3H, m), 4.79 (2H, q), 5.23 (1H, s), 6.65 (1H, s), 8.63 (1H, d), 8.82 (1H, d), 10.57 (1H, s).

### b) 4-[2-Ethoxv-5-(4-ethylpiperazin-1-ylsulphonyl)pyridin-3-ylcarboxamido]-3-ethyl-2-(1-methylpiperidin-4-yl)pyrazole-5-carboxamide

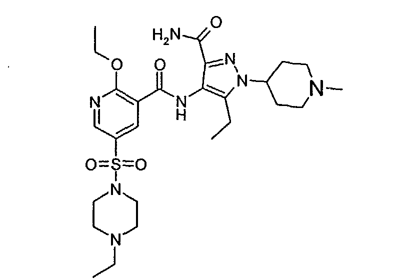

Trifluoroacetic acid (1.5ml) was added to a solution of the product from stage a) above (320mg, 0.48mmol) in dichloromethane (2ml) and the solution stirred at room temperature for 2 ½ hours. The reaction mixture was evaporated under reduced pressure and the residue triturated well with ether and dried under vacuum, to provide a white solid. Formaldehyde (217 microlitres, 37% aqueous, 2.90mmol) was added to a solution of the intermediate amine in dichloromethane (8ml), and the solution stirred vigorously for 30 minutes. Acetic acid (88 microlitres, 1.69mmol) was added, the solution stirred for a further 30 minutes, then sodium triacetoxyborohydride (169mg, 0.80mmol) was added and the reaction stirred at room temperature for 16 hours. The reaction mixture was poured into aqueous sodium bicarbonate solution, and extracted with ethyl acetate. The combined organic extracts were dried (MgSO₄) and evaporated under reduced pressure. The residue was purified by column chromatography on silica gel using dichloromethane:methanol:0.88 ammonia (91.75:7.5:0.75) as eluant to afford the title compound, 70mg; δ (CDCl₃) 1.02 (3H, t), 1.22 (3H, t), 1.58 (3H, t), 1.92 (2H, m), 2.14 (2H, m), 2.25-2.45 (7H, m), 2.54 (4H, m), 2.91 (2H, q), 2.99-3.16 (6H, m), 4.08 (1H, m), 4.78 (2H, q), 5.11 (1H, br s), 6.65 (1H, br s), 8.63 (1H, d), 8.83 (1H, d), 10.53 (1H, s).

### cGMP Example 3

### 5-[2-Ethoxv-5-(4-ethvlpiperazin-1-ylsulphonyl)pyridin-3-yl]-3-ethvl-2-phenvl-2,6-dihydro-7H-pyrazolo[4,3-d]pyrimidin-7-one

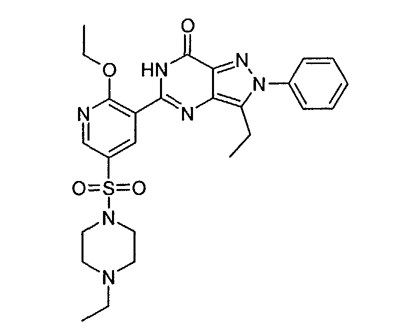

Pyridine (0.1 ml, 1.08mmol) was added to a mixture of the product from stage a) below (250mg, 0.54mmol), copper (II) acetate monohydrate (145mg, 0.72mmol), benzeneboronic acid (132mg, 1.08mmol) and 4Å molecular sieves (392mg) in dichloromethane (5ml), and the reaction stirred at room temperature for 4 days. The reaction mixture was filtered and the filtrate evaporated under reduced pressure. The crude product was purified by column chromatography on silica gel using dichloromethane:methanol:0.88 ammonia (97:3:0.5) as eluant, and triturated with ether:hexane. The resulting solid was filtered and recrystallised from *iso*-propanol:dichloromethane to give the title compound as a solid, 200mg, δ (CDCl₃) 1.02 (3H, t), 1.47 (3H, t), 1.60 (3H, t), 2.42 (2H, q), 2.58 (4H, m), 3.10 (2H, q), 3.17 (4H, m), 4.76 (2H, q), 7.40 (1H, m), 7.51 (2H, m), 7.80 (2H, d), 8.67 (1H, d), 9.16 (1H, s), 10.90 (1H, s); LRMS : m/z 538 (M+1)⁺.

### Preparation of Starting Materials

### a) 5-[2-Ethoxy-5-(4-ethylpiperazin-1-ylsulphonyl)pyridin-3-yl]-3-ethyl-2,6-dihydro-7H-pyrazolo[4,3-d]pyrimidin-7-one

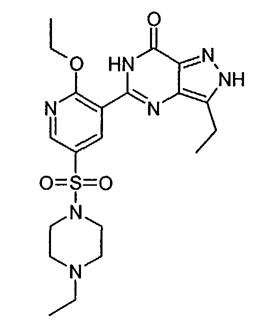

Potassium bis(trimethylsilyl)amide (8.28g, 41.6mmol) was added to a solution of the product from Example 1, stage h) (10.0g, 20.8mmol) and ethyl acetate (2ml, 20mmol) in ethanol (160ml), and the reaction mixture heated at 120°C for 12 hours in a sealed vessel. The cooled mixture was evaporated under reduced pressure and the residue was purified by column chromatography on silica gel using dichloromethane:methanol:0.88 ammonia (95:5:0.5) as eluant, to give the title compound, 3.75g; δ (CDCl₃) 1.03 (3H, t), 1.42 (3H, t), 1.60 (3H, t), 2.42 (2H, q), 2.58 (4H, m), 3.02 (2H, q), 3.16 (4H, m), 4.78 (2H, q), 8.66 (1H, d), 9.08 (1H, d), 11.00 (1 H, s) 11.05-11.20 (1 H, br s), LRMS : m/z 462 (M+1)⁺.

### cGMP Example 4

### 5-(5-Acetvl-2-propoxv-3-pyridinyl)-3-ethvl-2-(1-isopropyl-3-azetidinyl)-2,6-dihvdro-7H-pyrazolo[4,3-d]pyrimidin-7-one

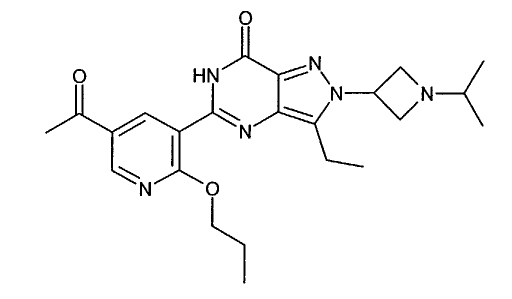

The product from stage h) below (0.23 mmol) was dissolved in dichloromethane (10 ml) and acetone (0.01 ml) was added. After 30 min stirring sodium triacetoxyborohydride (0.51 mmol) was added and stirring continued for 14 h. Further acetone (0.01 ml) and sodium triacetoxyborohydride (0.51 mmol) were added and stirring continued for a further 4.5 h. Starting material still remained so further acetone (0.01 ml) and sodium triacetoxyborohydride (0.51 mmol) were added and stirring continued for a further 18 h. The reaction mixture was diluted with dichloromethane, washed with sodium bicarbonate solution then brine, dried (MgSO₄) and concentrated. Purification by flash column chromatography (elution with 94:6:0.6 dichloromethane/methanol/0.88 ammonia) gave the product as a solid, M.p. 162.8-163.6°C; 1H NMR (400MHz, MeOD): δ = 1.00 (app. d, 9H), 1.30 (t, 3H), 1.84 (app. q, 2H), 2.60 (s, 3H), 2.62-2.72 (m, 1H), 3.00-3.10 (q, 2H), 3.75 (t, 2H), 3.90 (t, 2H), 4.50 (t, 2H), 5.25 (t, 1H), 8.70 (s, 1H), 8.90 (s, 1H); LRMS (TSP - positive ion) 439 (MH⁺); Anal. Found C, 61.92; H, 6.84; N, 18.70 Calcd for C₂₃H₃₀O₃N₆.0.1CH₂Cl₂: C, 62.07; H, 6.81; N, 18.80.

### Preparation of Starting Materials

### a) 2-Propoxy-5-iodonicotinic acid

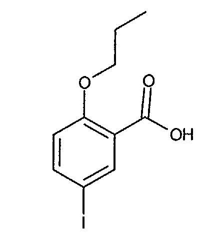

*N*-Iodosuccinamide (18.22 g, 0.08 mol), trifluoroacetic acid (100 ml) and trifluoroacetic anhydride (25 ml) were added to 2-propoxynicotinic acid (0.054 mol). The mixture was refluxed for 2.5 h, cooled and the solvents evaporated. The residue was extracted from water with ethyl acetate and the organics washed with water (twice) and brine (twice), dried (MgSO₄) and concentrated. The red residue was redissolved in ethyl acetate washed with sodium thiosulfate solution (twice), water (twice), brine (twice), redried (MgSO₄) and concentrated to give the desired product as a solid; ¹H NMR (300 MHz, CDCl₃): δ = 1.05 (t, 3H), 1.85-2.0 (m, 2H), 4.5 (t, 2H), 8.5 (s, 1H), 8.6 (s, 1H); Analysis: found C, 35.16; H, 3.19; N, 4.46. Calcd for C₉H₁₀INO₃: C, 35.19; H, 3.28; N, 4.56%.

### b) N-[3-(Aminocarbonyl)-5-ethyl-1H-pyrazol-4-yl]-5-iodo-2-propoxy-nicotinamide

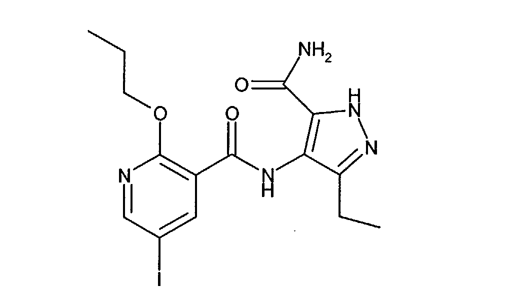

Oxalyl chloride (15.9 mmol) was added to a stirred solution of the product from stage a) (3.98 mmol) in dichloromethane (20 ml) and 3 drops *N,N*-dimethylformamide added. After 2.5 h the solvent was evaporated and the residue azeotroped 3 times with dichloromethane. The residue was resuspended in dichloromethane (4 ml) and added to a stirred mixture 4-amino-3-ethyl-1 *H*-pyrazole-5-carboxamide (prepared as described in WO 98/49166) (3.58 mmol) and triethylamine (7.97 mmol) in dichloromethane (10 ml). After 1 h the solvent was evaporated and the residue partitioned between ethyl acetate and water. The organic phase was separated and washed with 2N HCI (twice), sodium bicarbonate solution (twice) and brine before being dried (MgSO₄) and concentrated. The product was triturated with ether and filtered to give the title product as a solid. The mother liquor was concentrated and purified by flash column chromatography (elution with 80% ethyl acetate : hexane) to give further product; ¹H NMR (300 MHz, d₄-MeOH): δ = 1.0 (t, 3H), 1.25 (t, 3H), 1.85-2.0 (m, 2H), 2.8 (q, 2H), 4.5 (t, 2H), 8.5 (s, 1H), 8.6 (s, 1H); LRMS (TSP) 444 (MH⁺).

### c) tert-Butyl 3-iodo-1-azetidinecarboxylate

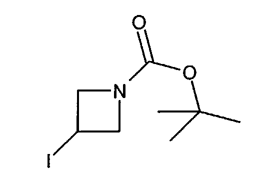

A mixture of *tert*-butyl 3-[(methylsulfonyl)oxy]-1-azetidinecarboxylate (prepared as described in *Synlett* 1998, 379; 5.0 g, 19.9 mmol), and potassium iodide (16.5 g, 99.4 mmol) in *N,N*-dimethylformamide (25 ml), was heated at 100°C for 42 h. The cooled mixture was partitioned between water and ethyl acetate, and the layers separated. The organic phase was dried over MgSO₄, concentrated under reduced pressure and the residue azeotroped with xylene. The crude product was purified by flash column chromatography (dichloromethane as eluant) to give the title compound, 3.26 g; ¹H NMR (300 MHz, CDCl₃) δ = 1.43 (s, 9H), 4.28 (m, 2H), 4.46 (m, 1H), 4.62 (m, 2H); LRMS (TSP) 284 (MH)⁺

### d) tert-Butyl 3-(3-(aminocarbonvl)-5-ethyl-4-{[(5-iodo-2-propoxy-3-pyridinyl)carbonyl]amino}-1H-pyrazol-1-yl)-1-azetidinecarboxylate

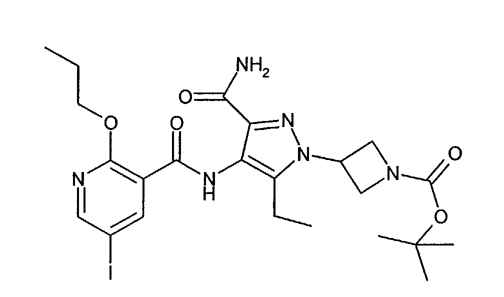

Cesium carbonate (3.59 mmol) was added to a stirred solution of the product from stage b) (1.79 mmol) and the product from stage c) (2.15 mmol) in *N,N*-dimethylformamide (10 ml) under a nitrogen atmosphere. The mixture was heated at 80°C for 24 h. The mixture was cooled and extracted from water with ethyl acetate. The organics were dried (MgSO₄) and concentrated to give a brown oil. Purification by flash column chromatography (gradient elution from 100% dichloromethane to 90% dichloromethane/MeOH) gave the title product; 1H NMR (400MHz, DMSO): δ = 0.95 (t, 3H), 1.05 (t, 3H), 1.40 (s, 9H), 1.78-1.88 (m, 2H), 2.68 (q, 2H), 4.22-4.35 (m, 4H), 4.40 (t, 2H), 5.33 (t, 1H), 7.35 (bs, 1H), 7.52 (bs, 1H), 8.40 (s, 1H), 8.55 (s, 1H), 10.10 (s, 1H); LRMS (TSP - positive ion) 373.2 (MH⁺ - BOC and I); Anal. Found C, 45.11; H, 5.07; N, 13.56 Calcd for C₂₃H₃₁O₅N₆I. 0.2 DCM: C, 45.28; H, 5.14; N, 13.66.

### e) tert-Butyl 3-[3-ethyl-5-(5-iodo-2-propoxy-3-pyridinyl)-7-oxo-6,7-dihydro-2H-pyrazolo[4,3-d]pyrimidin-2-yl]-1-azetidinecarboxylate

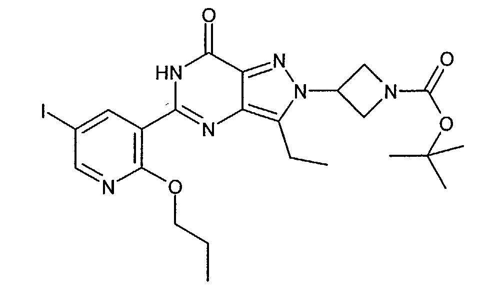

The product from stage d) (28.4 mmol) was dissolved in n-propanol (200 ml), ethyl acetate (6 ml) and potassium t-butoxide (28.4 mmol) were added and the resultant mixture heated to reflux for 6h. Additional potassium t-butoxide (14.2 mmol) was added and the mixture heated for a further 2h, after which the solvent was removed *in vacuo.* The residue was partioned between water (50 ml) and methylene chloride (100 ml) and the organic phase separated. The aqueous phase was extracted with dichloromethane (2 x 100 ml) and the combined organics dried over MgSO₄ and reduced to a solid. Purification by column chromatography (elution with ethyl acetate) gave the title compound; 1H NMR (400MHz, CDCl₃): δ = 1.05 (t, 3H), 1.30 (t, 3H), 1.43 (s, 9H), 1.87-1.96 (m, 2H), 3.00 (q, 2H), 4.34 (t, 2H), 4.49 (t, 2H), 4.60 (br s, 2H), 5.20 (t, 1H), 8.41 (d, 1H), 8.94 (s, 1H), 10.75 (br s, 1H); LRMS (TSP - positive ion) 598.1 (MNH₄⁺); Anal. Found C, 47.54; H, 5.02; N, 14.09 Calcd for C₂₃H₂₉O₄N₆I: C, 47.60; H, 5.04; N, 14.48.

### f) tert-Butyl 3-(3-ethyl-7-oxo-5-{2-propoxy-5-[(trimethylsilyl)ethynyl]-3-pyridinyl}-6,7-dihydro-2H-pyrazolo[4,3-d]pyrimidin-2-yl)-1-azetidinecarboxylate

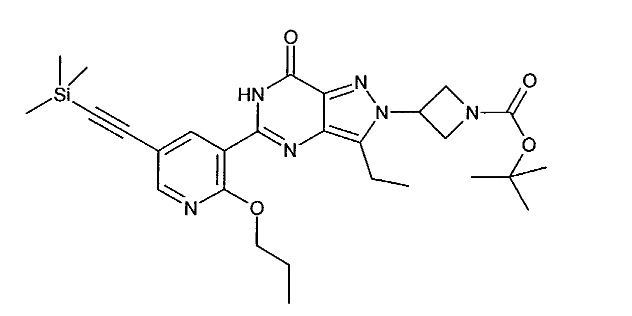

The product from stage e) (0.25 mmol) was suspended in triethylamine (2 ml) and trimethylsilylacetylene (0.39 mmol) and acetonitrile (2 ml to try and solubilise reactants). Pd(PPh₃)₂Cl₂ (0.006 mmol) and cuprous iodide (0.006 mmol) were added and the reaction mixture stirred. After 1 h a further portion of trimethylsilylacetylene (0.19 mmol) was added and stirring continued for 2 h. The solvent was evaporated and the residue partitioned between ethyl acetate and water. The organics were washed with brine, dried (MgSO₄) and concentrated. Purification by flash column chromatography (gradient elution from 100% dichloromethane to 99% dichloromethane/methanol) gave the title compound; 1H NMR (400MHz, MeOD): δ = 0.25 (s, 9H), 1.05 (t, 3H), 1.31 (t, 3H), 1.44 (s, 9H), 1.87-1.96 (m, 2H), 3.00 (q, 2H), 4.33 (t, 2H), 4.52 (t, 2H), 4.54-4.80 (m, 2H), 5.18-5.25 (m, 1H), 8.32 (d, 1H), 8.74 (d, 1H); LRMS (TSP - positive ion) 569 (MNH₄⁺), 452.0 (MH⁺); Anal. Found C, 60.82; H, 6.90; N, 15.15 Calcd for C₂₈H₃₈O₄N₆Si: C, 61.07; H, 6.95; N, 15.26.

### g) tert-Butyl 3-[3-ethyl-5-(5-ethynyl-2-propoxy-3-pyridinyl)-7-oxo-6,7-dihydro-2H-pyrazolo[4,3-d]pyrimidin-2-yl]-1-azetidinecarboxylate

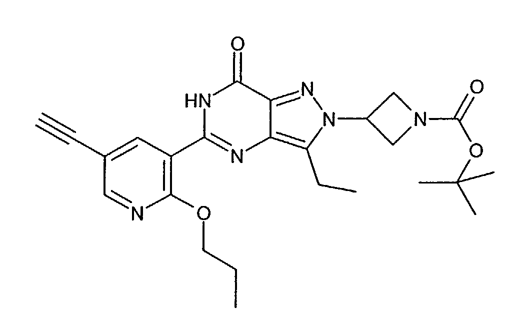

Potassium fluoride (0.38 mmol) was added to a stirred solution of the product of stage f) (0.19 mmol) in aqueous *N*,*N*-dimethylformamide (2 ml *N,N*-dimethylformamide /0.2 ml water) at 0°C. After 10 min the reaction was allowed to warm to room temperature and stirred for 2 h. The reaction mixture was diluted with ethyl acetate and washed with water, 1 *N* hydrochloric acid (3 times) and brine. The organic layer was dried (MgSO₄) and concentrated to give the title compound as a solid; 1H NMR (400MHz, CDCl₃): δ = 1.05 (t, 3H), 1.30 (t, 3H), 1.43 (s, 9H), 1.88-2.00 (m, 2H), 3.00 (q, 2H), 3.19 (s, 1H), 4.35 (app t, 2H), 4.52 (app t, 2H), 4.60-4.80 (br s, 2H), 5.22 (t, 1H), 8.39 (s, 1H), 8.80 (s, 1H), 10.75 (br s, 1H); LRMS (TSP - positive ion) 496 (MNH₄⁺).

### h) 5-(5-Acetyl-2-propoxy-3-pyridinyl)-2-(3-azetidinyl)-3-ethyl-2,6-dihydro-7H-pyrazolo[4,3-d]pyrimidin-7-one

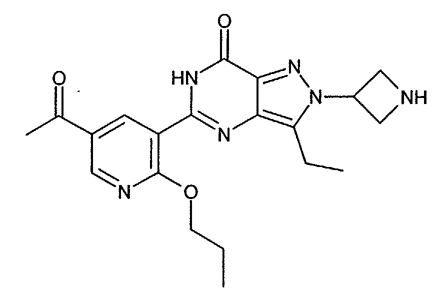

The product from stage g) (1.44 g, 3.0 mmol) in acetone (50 ml) and sulphuric acid (1N, 3 ml) was treated with mercuric sulphate (268 mg, 9.0 mmol) and heated to reflux for 6h. The reaction mixture was concentrated to ∼20 ml *in vacuo,* poured into sodium bicarbonate (sat. aq., 20ml) and extracted into methylene chloride (6 x 20 ml). Combined organics were washed with brine (20 ml), dried over MgSO₄, and concentrated to a brown oil which was taken up in 40% trifluoroacetic acid in methylene chloride (50ml) and water (1 ml) and stirred for 1h at room temperature. After evaporation *in vacuo*, the residue was purified by column chromatography (eluting with 95:5:1 methylene chloride:methanol:0.88 ammonia) to afford the title compound as a white hydroscopic foam (1.65 g); m.p. 128.5-130.0°C; 1H NMR (400MHz, MeOD): δ = 1.00 (t, 3H), 1.30 (t, 3H), 1.79-1.90 (m, 2H), 2.60 (s, 3H), 3.00-3.10 (q, 2H), 4.50 (t, 2H), 4.60-4.70 (m, 4H), 5.65-5.78 (m, 1H), 8.65 (s, 1H), 8.90 (s, 1H); LRMS (TSP - positive ion) 397 (MH⁺).

### cGMP Example 5

### 5-(5-Acetvl-2-butoxv-3-pyridinyl)-3-ethyl-2-(1-ethvl-3-azetidinyl)-2,6-dihydro-7H-pyrazolo[4,3-d]pyrimidin-7-one

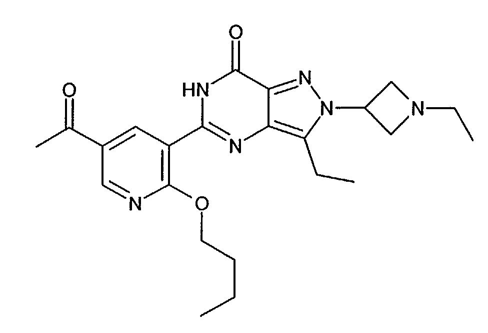

The starting material (120 mg, 0.28 mmol) and cesium carbonate (274 mg, 0.84 mmol) were dissolved in n-butanol (4 ml), and heated at 90°C under nitrogen with molecular sieves for 96h. The mixture was then partitioned between water (10 ml) and dichloromethane (10 ml). The organic layer was separated, and the aqueous layer extracted further with dichloromethane (3 x 15 ml). The combined organic layers were dried (MgSO₄), and concentrated *in vacuo.* The crude product was purified by flash column chromatography (95:5:0.5-90:10:1 ethyl acetate:methanol:0.88 NH₃ as eluents), to yield the title compound as a colourless glass (77 mg, 0.18 mmol); m.p. 91.6-93.7°C; 1H NMR (400MHz, CDCl₃): δ = 1.00-1.05 (m, 6H), 1.38 (t, 3H), 1.50-1.62 (m, 2H), 1.90-2.00 (m, 2H), 2.63 (s, 3H), 2.63-2.70 (m, 2H), 3.02 (q, 2H), 3.75 (t, 2H), 3.90 (t, 2H), 4.68 (t, 2H), 5.10-5.20 (m, 1H), 8.84 (s, 1H), 9.23 (s, 1H), 10.63 (br s, 1H); LRMS (TSP - positive ion) 439 (MH⁺); Anal. Found C, 60.73; H, 7.06; N, 18.03 Calcd for C₂₃H₃₀O₃N₆.0.2MeOH.0.1 DIPE: C, 60.88; H, 7.26; N, 17.90.

### Preparation of Starting Materials

### 5-(5-Acetvl-2-propoxy-3-pyridinyl)-3-ethvl-2-(1-ethvl-3-azetidinvl)-2,6-dihvdro-7H-pyrazolo[4,3-d]pyrimidin-7-one

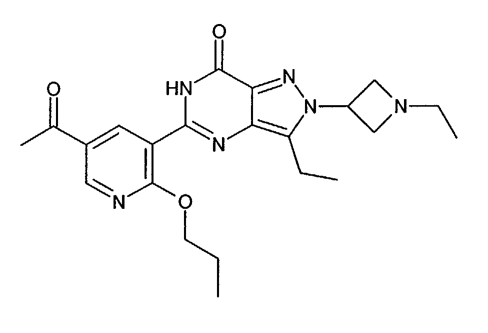

Sodium cyanoborohydride (92 mg, 1.47 mmol) was added to a stirred solution of the product from Example 4 stage h) (500 mg, 0.98 mmol) and sodium acetate (161 mg, 1.96 mmol) in methanol (10 ml) under nitrogen at room temperature. After 1h the mixture was poured into NaHCO₃ (sat. aq., 20 ml), and extracted with dichloromethane (3 x 15 ml). The combined organic layers were dried (MgSO₄) and concentrated *in vacuo.* The crude product was purified by flash column chromatography (95:5:0.5-80:20:1 ethyl acetate:methanol:0.88 NH₃ as eluent) to yield the title compound as a white solid (140 mg, 0.33 mmol); 1H NMR (400MHz, CDCl₃): δ = 0.97 (t, 3H), 1.03 (t, 3H), 1.30 (t, 3H), 2.82-2.97 (m, 2H), 2.58-2.65 (m, 5H), 2.98 (q, 2H), 3.68 (t, 2H), 3.85 (dd, 2H), 4.58 (dd, 2H), 5.05-5.17 (m, 1H), 8.79 (s, 1H), 9.18 (s, 1H), 10.62 (br s, 1H); LRMS (TSP - positive ion) 426 (MH⁺).

Oral daily dosages of the above cGMP elevators can range from about 1 mg to about 200 mg with a preferred range of from about 20 mg to about 100 mg. Dosage is *ad libitum* from about 15 minutes to about 4 hours prior to sexual activity. Dosages and timing of dosing can be adjusted for topical dosage forms such as creams or aerosols. cGMP elevators of the present invention include produgs, stereoisomers, hydrates, tautomers and salts of the described compounds. The cGMP elevators of the present invention may be formulated and administered as described for the estrogen agonists / antagonists above.

The cGMP PDE inhibitors useful in this invention as cGMP elevators may be chosen from among any of those already known to the art or subsequently discovered and/or hereafter developed. Suitable cGMP PDE inhibitors include those disclosed in any of the following US patents:
a 5-substituted pyrazolo[4,3-d]pyrimidine-7-one as disclosed in US 4,666,908;
a griseolic acid derivative as disclosed in any of US 4,634,706, 4,783,532, 5,498,819, 5,532,369, 5,556,975, and 5,616,600;
a 2-phenylpurinone derivative as disclosed in US 4,885,301;
a phenylpyridone derivative as disclosed in US 5,254,571;
a fused pyrimidine derivative as disclosed in US 5,047,404;
a condensed pyrimidine derivative as disclosed in US 5,075,310;
a pyrimidopyrimidine derivative as disclosed in US 5,162,316;
a purine compound as disclosed in US 5,073,559;
a quinazoline derivative as disclosed in US 5,147,875;
a phenylpyrimidone derivative as disclosed in US 5,118,686;
an imidazoquinoxalinone derivative or its aza analog as disclosed in US 5,055,465 and 5,166,344;
a phenylpyrimidone derivative as disclosed in US 5,290,933;
a 4-aminoquinazoline derivative as disclosed in US 5,436,233 or 5,439,895;
a 4,5-dihydro-4-oxo-pyrrolo[1,2-a]quinoxaline derivative as disclosed in US 5,405,847;
a polycyclic guanine derivative as disclosed in US 5,393,755;
a nitogenous heterocyclic compound as disclosed in US 5,576,322;
a quinazoline derivative as disclosed in US 4,060,615;
a 6-heterocyclyl pyrazolo[3,4-d]pyrimidin-4-one as disclosed in US 5,294,612; and
a 4-aminoquinazoline derivative as disclosed in US 5,436,233;

Other disclosures of cGMP PDE inhibitors include the following, all of which are herein incorporated by reference:
European patent Application (EPA) publication no. 0428268;
European patent 0442204;
International patent application publication no. WO 94/19351;
Japanese patent application 5-222000;
European Journal of Pharmacology, 251, (1994), 1;
International patent application publication no. WO 94/22855;
a pyrazolopyrimidine derivative as disclosed in European patent application 0636626;
a 4-aminopyrimidine derivative as disclosed in European patent application 0640599;
an imidazoquinazoline derivative as disclosed in International patent application WO95/06648;
an anthranilic acid derivative as disclosed in International patent application WO95/18097;
a tetracyclic derivative as disclosed in International patent application WO95/19978;
an imidazoquinazoline derivative as disclosed in European patent application 0668280; and
a quinazoline compound as disclosed in European patent application 0669324.

The cGMP PDE inhibition of a compound can be determined by standard assays known to the art, for example as disclosed in US 5,250,534. Compounds which are selective inhibitors of cGMP PDE relative to cAMP PDE are preferred, and determination of such compounds is also taught in US 5,250,534. Particularly preferred are compounds which selectively inhibit the PDE_{V} isoenzyme, as disclosed in the aforementioned PCT/EP94/01580, published as WO 94/28902.

It will be recognized that certain of the above cGMP elevators contain either a free carboxylic acid or a free amine group as part of the chemical structure. Further, certain cGMP elevators within the scope of this invention contain lactone moieties, which exist in equilibrium with the free carboxylic acid form. These lactones can be maintained as carboxylates by preparing pharmaceutically acceptable salts of the lactone. Thus, this invention includes pharmaceutically acceptable salts of those carboxylic acids or amine groups.

The expression "pharmaceutically acceptable salts" includes both pharmaceutically acceptable acid addition salts and pharmaceutically acceptable cationic salts. The expression "pharmaceutically-acceptable cationic salts" is intended to define but is not limited to such salts as the alkali metal salts, (e.g. sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts. It will also be recognized that it is possible to administer amorphous forms of the cGMP elevators.

The pharmaceutically-acceptable cationic salts of cGMP elevators containing free carboxylic acids may be readily prepared by reacting the free acid form of the cGMP elevator with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine and tromethamine. The salt is isolated by concentration to dryness or by addition of a non-solvent. In many cases, salts are preferably prepared by mixing a solution of the acid with a solution of a different salt of the cation (e.g., sodium or potassium ethylhexanoate, magnesium oleate), employing a solvent (e.g., ethyl acetate) from which the desired cationic salt precipitates, or can be otherwise isolated by concentration and/or addition of a non-solvent.

The pharmaceutically acceptable acid addition salts of cGMP elevators containing free amine groups may be readily prepared by reacting the free base form of the cGMP elevator with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent.

One of ordinary skill in the art will recognize that when the compounds identified by the present invention are used in combination with certain estrogen agonists / antagonists, described above, or with certain cGMP elevators, as described above, the estrogen agonists/ antagonists or cGMP elevators will contain one or more atoms which may be in a particular stereochemical, tautomeric, or geometric configuration, giving rise to stereoisomers, tautomers and configurational isomers. All such isomers and mixtures thereof are included in this invention. Hydrates and solvates of the compounds of this invention are also included.

The subject invention also includes isotopically-labeled estrogen agonists /antagonists or cGMP elevators, which may be used in combination with the compounds identified in the present invention, and which are structurally identical to those disclosed above, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, prodrugs thereof, and pharmaceutically acceptable salts of said compounds and of said prodrugs which contain the aforementioned isotopes and/or other isotopes of other atoms are within the scope of this invention.

Pharmaceutical chemists will easily recognize that physiologically active compounds which have accessible hydroxy groups are frequently administered in the form of pharmaceutically acceptable esters. The literature concerning such compounds, such as estradiol, provides a great number of instances of such esters. The compounds of this invention are no exception in this respect, and can be effectively administered as an ester, formed on the hydroxy groups, just as one skilled in pharmaceutical chemistry would expect. It is possible, as has long been known in pharmaceutical chemistry, to adjust the rate or duration of action of the compound by appropriate choices of ester groups.

Certain ester groups are preferred as constituents of the compounds of this invention. As noted above, the compounds identified by the present invention may be used in combination with estrogen agonists / antagonists or cGMP elevators, including the compounds of formula I, IA, II, III, IV, V, Va, VI, VII, VIII, IX, X, XI or Xia, which may contain ester groups at various positions as defined herein above, where these groups are represented as -COOR, R is C₁ -C₁₄ alkyl, C₁ -C₃ chloroalkyl, C₁ -C₃ fluoroalkyl, C₅ -C₇ cycloalkyl, phenyl, or phenyl mono- or disubstituted with C₁ -C₄ alkyl, C₁-C₄ alkoxy, hydroxy, nitro, chloro, fluoro or tri(chloro or fluoro)methyl.

The specific dose of a compound administered according to this invention will, of course, be determined by the particular circumstances surrounding the case including, for example, the compound administered, the route of administration, the state of being of the patient, and the severity of the pathological condition being treated. The dose of a compound of this invention to be administered to a subject is rather widely variable and subject to the judgement of the attending physician. It should be noted that it may be necessary to adjust the dose of a compound when it is administered in the form of a salt, such as a laureate, the salt forming moiety of which has an appreciable molecular weight.

The following dosage amounts and other dosage amounts set forth elsewhere in this description and in the appendant claims are for an average human subject having a weight of about 65 kg to about 70 kg. The skilled practitioner will readily be able to determine the dosage amount required for a subject whose weight falls outside the 65 kg to 70 kg range, based upon the medical history of the subject and the presence of diseases, e.g., diabetes, in the subject. All doses set forth herein, and in the appendant claims, are daily doses of the free base form of the estrogen agonists / antagonists or cGMP elevators. Calculation of the dosage amount for other forms of the free base form such as salts or hydrates is easily accomplished by performing a simple ratio relative to the molecular weights of the species involved.

The general range of effective administration rates of the estrogen agonists / antagonists is from about 0.001 mg/day to about 200 mg/day. A preferred rate range is from about 0.010 mg/day to 100 mg/day. Of course, it is often practical to administer the daily dose of compound in portions, at various hours of the day. However, in any given case, the amount of compound administered will depend on such factors as the potency of the specific estrogen agonist/antagonist, the solubility of the compound, the formulation used and the route of administration.

For the treatment of female sexual dysfunction, an estrogen and, optionally a progestin, may be coadministered with a compound identified by the present invention either separately or in a single composition.

Estrogens useful in the combinations of this invention include estrone, estriol, equilin, estradiene, equilenin, ethinyl estradiol, 17β-estradiol, 17α-dihydroequilenin, 17β-dihydroequilenin (U.S. Patent 2,834,712), 17α-dihydroequilin, 17β-dihydroequilin, menstranol and conjugated estrogenic hormones, such as those in Wyeth-Ayerst Laboratories' Premarin® products. Phytoestrogens, such as equol or enterolactone, may also be used in the present formulations and methods of the present invention. Esterified estrogens, such as those sold by Solvay Pharmaceuticals, Inc. under the Estratab® tradename, may also be used in the present combinations. Also preferred for use in the present invention are the salts of the applicable estrogens, most preferably the sodium salts. Examples of these preferred salts are sodium estrone sulfate, sodium equilin sulfate, sodium 17alpha-dihydroequilin sulfate, sodium 17alpha-estradiol sulfate, sodium delta8,9-dehydroestrone sulfate, sodium equilenin sulfate, sodium 17beta-estradiol sulfate, sodium 17beta-dihydroequilenin sulfate, estrone 3-dosium sulfate, equilin 3-sodium sulfate, 17alpha-dihydroequilin 3-sodium sulfate, 3beta-Hydroxy-estra-5(10),7-dien-17-one 3-sodium sulfate, 5alpha-pregnan-3beta-20R-diol 20-sodium sulfate, 5alpha-pregnn-3beta,16alpha-diol-20-one 3-sodium sulfate, delta(8,9)-dehydroestrone 3-sodium sulfate, estra-3beta, 17alpha-diol 3-sodium sulfate, 3beta-Hydroxy-estr-5(10)-en-17-one 3-sodium sulfate or 5alpha-Pregnan-3beta,16alpha,20R-triol 3-sodium sulfate. Preferred salts of estrone include, but are not limited to, the sodium and piperate salts. Other salts are described below.

Another type of compound that is useful in the present invention are the synthetic steroids such as tibolone (Livial®). The combination of a compound identified by the present invention with tibolone for the treatment of female sexual dysfunction, such as to enhance libido, treat dyspareunia, treat sexual arousal disorder, treat hypoactive sexual desire disorder, treat vaginismus, or increase the frequency or intensity of orgasms is preferred. The use of tibolone and a compound identified by the present invention can also optionally include a progestin.

Progestins are familiar to those skilled in the art. Examples of specific progestins that can be used in the present invention include, but are not limited to, levonorgestrel, norethindrone, ethynodiol, desogestrel, norgestrel, norgestimate, and medroxyprogesterone. In pharmaceutical compositions, it is common to use a salt of the progestins, which salts are described below.

The pharmaceutically acceptable acid addition salts of the estrogens and progestins, if applicable, include salts formed with inorganic or organic acids such as hydrochloric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like, most preferable with hydrochloric acid, citric acid, benzoic acid, maleic acid, acetic acid and propionic acid.

These salts may be formed by reacting the compound with a suitable acid. The salts are frequently formed in high yields at moderate temperatures, and often are prepared by merely isolating the compound from a suitable acidic wash as the final step of the synthesis. The salt-forming acid is dissolved in an appropriate organic solvent, or aqueous organic solvent, such as an alkanol, ketone or ester. On the other hand, if the compound of this invention is desired in the free base form, it is isolated from a basic final wash step, according to the usual practice. A preferred technique for preparing hydrochlorides is to dissolve the free base in a suitable solvent and dry the solution thoroughly, as over molecular sieves, before bubbling hydrogen chloride gas through it. It will also be recognized that it is possible to administer amorphous forms of the estrogens and progestins.

One of ordinary skill in the art will recognize that certain estrogens or progestins will contain one or more atoms which may be in a particular stereochemical, tautomeric, or geometric configuration, giving rise to stereoisomers, tautomers and configurational isomers. All such isomers and mixtures thereof are included in this invention. Hydrates and solvates of the compounds of this invention are also contemplated.

An acceptable dosage range for estrogens includes, but is not limited to, about 0.001 mg/day to about 100 mg/day. A preferred estrogen dosage range includes, but is not limited to about 0.010 mg/day to about 2 mg/day. An acceptable dosage range for progestins includes, but is not limited to about 0.001 mg/day to about 1000 mg/day. A preferred dosage range for the progestins is about 0.1 mg/day to about 500 mg/day.

For the treatment of female sexual dysfunction, the compounds identified by the present invention may be used in combination with other active agents, such as the following:
Prostaglandins (see, e.g., J.L. Yeager et al., "Prostaglandin-containing compositions and methods for amelioration of human female sexual dysfunction," PCT International Application WO 0033825 (2000));
Apomorphine;
Oxytocin modulators (see, e.g., T. Smock and P. Stark, "A peptidergic basis for sexual behavior in mammals," Prog. Brain Res. (1998), 119 (Advances in Brain Vasopressin), 467-481; see also A. Argiolas, "Neuropeptides and sexual behavior," Neurosci. Biobehav. Rev. (1999), 23(8), 1127-1142);
α-2 Adrenergic antagonists, such as yohimbine and rauwolscine (see, e.g., C.M. Meston et al., "Inhibition of subjective and physiological sexual arousal in women by clonidine," Psychosom. Med. (1997), 59(4), 399-407);
Androgens and selective androgen receptor modulators (SARMs) (see, e.g., S.H.M. Van Goozen et al., "Gender differences in behavior: activating effects of cross-sex hormones," Psychoneuroendocrinology (1995), 20(4), 343-63);
bupropion (Zyban®) (combined norepinephrine/dopamine (NE/DA) reuptake blocker);
Vasoactive intestinal peptide (VIP) (see, e.g., B. Ottesen et al.,"Vasoactive intestinal polypeptide (VIP) provokes vaginal lubrication in normal women," Peptides (1987), 8(5), 797-800);
Neutral endopeptidase inhibitors (NEP) (see, e.g., G.N. Maw and C.P. Wayman, "NEP (neutral endopeptidase) inhibitors for the treatment of female sexual dysfunction," Eur. Pat. Appl. 1097719 (2001)); and
Neuropeptide Y receptor antagonists (NPY) (see, e.g., G.N. Maw and C.P. Wayman, "Neuropeptide Y (NPY) antagonists for the treatment of female sexual dysfunction," Eur. Pat. Appl. 1097718 (2001)).

In one embodiment, the present invention provides a method of treating female sexual dysfunction, the method comprising the step of administering to a female patient in need thereof a therapeutically effective amount of a compound that attenuates the binding of agouti-related protein to melanocortin receptors, but does not attenuate the binding of α-melanocyte stimulating hormone to melanocortin receptors in combination with a compound that is a melanocortin receptor agonist.

Particularly preferred melanocortin receptor agonists are melanocortin-4 receptor agonists. Examples of melanocortin-4 receptor agonists include melanotan II (MT II), α-MSH and NDP-MSH.

An example of a compound that attenuates the binding of agouti-related protein to melanocortin receptors, particularly melanocortin-4 receptors, but does not attenuate the binding of α-melanocyte stimulating hormone to melanocortin receptors is 8,16-bis-(4-nitro-phenyl)-5,6,8,8a,13,14,16,16a-octahydro-[1,2,4,5]tetrazino[6,1-a;3,4-a']diisoquinoline, which can be synthesized in accordance with Deyrup, J. *et al., Tetrahedron Letters,* 24, 2191-2 (1971), or Grashey, R. *et al., Angew*. *Chem,* 74(8): 292-293 (1962).

All documents cited in this patent application are hereby incorporated by reference.

The examples presented below are intended to illustrate particular embodiments of the invention, and are not intended to limit the scope of the specification, including the claims, in any manner.

### Examples

In order to identify compounds that attenuate the binding of AGRP to melanocortin receptors, particularly melanocortin 3 and/or 4 receptors, but do not attenuate the binding of α-MSH to melanocortin receptors, a series of two radioligand binding assays can be used. The first radioligand binding assay is an [¹²⁵I]AGRP competition binding assay. Compounds that attenuate binding of [¹²⁵I]AGRP or AGRP would be detected in this assay. It is noted that compounds can attenuate the binding of AGRP to melanocortin 3 and/or 4 receptors by binding to melanocortin 3 and/or 4 receptors or by binding to AGRP itself. The second radioligand binding assay is an assay using radiolabeled melanocortin ligands, for example, [¹²⁵I]norleucine D-phenylalanine melanocyte stimulating hormone ([¹²⁵I]NDP-MSH). Membranes prepared from cells expressing either melanocortin 3 or 4 receptors are used in the radioligand binding assays. The preparation and use of [¹²⁵I]AGRP and [¹²⁵I]NDP-MSH is well known in the art. See, for example, Dang *et al., Molecular Endocrinology,* **13,** 148-155 (1999). In addition, the preparation and use of membranes from cells expressing either melanocortin 3 or 4 receptors are also well known in the art. See, Bass, *et al., Molecular Pharmacology,* **50,** 709-715 (1996).

### EXAMPLE 1 RADIOLIGAND BINDING ASSAYS

### [¹²⁵I]AGRP COMPETITION BINDING ASSAY

Specific Activity of [¹²⁵I]AGRP is 2200 Ci/mmole. The final concentration of [¹²⁵I]AGRP is 100 pM. Therefore, a 2 nM (20X) stock needs to be made in binding buffer. The concentration of [¹²⁵I]AGRP varies from 40-60 nM. [¹²⁵I]AGRP can be obtained from New England Nuclear, Boston, MA.

The competition assay can be run using 96 well plates. The last row (Row H) in the 96 well plate should be for total ("totals") counts per minute (cpm) bound (H1,2), 1 µM AGRP (H3,4), 1 µM NDP-MSH (H5,6) and filter blanks (just binding buffer buffer, no membranes; H7,8). The other rows (A-G) should be for compounds to be tested. Up to seven compounds can be tested in 7 point competition curves in a 96 well format. The first six rows for each compound can be used for testing 6 compounds at 6 concentrations in duplicate. An example for a single compound is outlined below. The next compound would be in rows A-F, columns 3 and 4. A seventh compound can be placed in row G1-12.

Samples are made in the following stock concentrations: 10⁻³, 10⁻⁴, 10⁻⁵, 10⁻⁶, 10⁻⁷, 10⁻⁸ M in binding buffer. The final concentrations will be one order of magnitude less (10⁻⁴ to 10⁻⁹). Stock concentration of compounds are usually 25 mM so a 25:1 dilution is required. Make up 6 tubes labeled -4 to -9. Put 100 µl of binding buffer in each tube. Add 4 µl of 25 mM stock to the tube labeled -4. Vortex and take 11 µl of the -4 sample and add to the -5 tube. Repeat until all the dilutions are made. For each row the compound will look like this:
A1,2 -9 (1nM)
B1,2 -8 (10nM)
C1,2 -7 (100nM)
D1,2 -6 (1µM)
E1,2 -5 (10µM)
F1,2 -4(100µM)

### To each well add in order:

20 µl of binding buffer to "total" wells (H1,2).
20 µl of 10 µM AGRP to wells H3,4.
20 µl of 10 µM NDP-MSH to wells H5,6.
170 µl of binding buffer to wells H7,8.
10 µl of 2 nM [¹²⁵I]AGRP to all wells.
170 µl membranes diluted to 5 µg/170 µl in binding buffer to all wells except H7,8.

### Procedure:

1. Set up assay for a 96 well filtering system (Unifilter® GF/C™, Packard Instrument Company, Meriden, CT).
2. Incubate 90-120 minutes shaking at room temperature.
3. Using a cell harvester, aspirate samples into processing head. Use a pre-soaked (0.3% polyethylene imine) filter.
4. Wash four times with cold wash buffer.
5. Dry plate, add 25 µl of scintillation fluid to each well.
6. Count samples.

### Binding Buffer:

50 mM Hepes/10 mM MgCl₂, pH 7.4 (made from 10X stock)
0.2 % BSA (fraction V)
Protease inhibitors (Made up as 100X stock).
100 µg/ml bacitracin
100 µg/ml benzamidine
5 µg/ml aprotinin
5 µg/ml leupeptin
The protease inhibitors can be obtained from Sigma, St. Louis, MO.

### Wash Buffer:

50 mM Hepes/10 mM MgCl₂, pH 7.4, ice cold (made from 10X stock).

### [¹²⁵I]NDP-MSH COMPETITION BINDING ASSAY

Specific Activity of [¹²⁵I]NDP-MSH is 2200 Ci/mmole. The final concentration of [¹²⁵I]NDP-MSH is 250 pM. Therefore, a 5 nM (20X) stock needs to be made in binding buffer. The concentration of [¹²⁵I]NDP-MSH varies from 40-60 nM. [¹²⁵I]NDP-MSH can be obtained from New England Nuclear, Boston, MA.

The competition assay can be run using 96 well plates. The last row (Row H) in the 96 well plate should be for total cpm bound (H1,2), 1 µM AGRP (H3,4), 1 µM NDP-MSH (H5,6) and filter blanks (just binding buffer, no membranes; H7,8). The other rows (A-G) should be for compounds to be tested. Up to seven compounds can be tested in 7 point competition curves in a 96 well format. The first six rows for each compound can be used for testing 6 compounds at 6 concentrations in duplicate. An example for a single compound is outlined below. The next compound would be in rows A-F, columns 3 and 4. A seventh compound can be placed in row G1-12.

Samples are made in the following stock concentrations: 10⁻³, 10⁻⁴, 10⁻⁵, 10⁻⁶, 10⁻⁷, 10⁻⁸ M in binding buffer. The final concentrations will be one order of magnitude less (10⁻⁴ to 10⁻⁹). Stock concentration of compounds are usually 25 mM so a 25:1 dilution is required. Make up 6 tubes labeled -4 to -9. Put 100 µl of binding buffer in each tube. Add 4 µl of 25 mM stock to the tube labeled -4. Vortex and take 11 µl of the -4 sample and add to the -5 tube. Repeat until all the dilutions are made. For each row the compound will look like this:
A1,2 -9
B1,2 -8
C1,2 -7
D1,2 -6
E1,2 -5
F1,2 -4

### To each well add in order:

20 µl binding buffer to "total" wells (H1,2).
20 µl of 10 µM AGRP to wells H3,4.
20 µl of 10 µM NDP-MSH to wells H5,6.
170 µl of binding buffer to wells H7,8.
10 µl of 2 nM [¹²⁵I]NDP-MSH to all wells.
170 µl membranes diluted to 5 µg/170 µl in binding buffer to all wells except H7,8.

### Procedure:

1. Set up assay for a 96 well filtering system (Unifilter® GF/C™, Packard Instrument Company, Meriden, CT).
2. Incubate 90-120 minutes shaking at room temperature.
3. Using a cell harvester, aspirate samples into processing head. Use a pre-soaked (0.3% polyethylene imine) filter.
4. Wash four times with cold wash buffer.
5. Dry plate, add 25 µl of scintillation fluid to each well.
6. Count samples.

### Binding Buffer:

50 mM Hepes/10 mM MgCl₂, pH 7.4 (made from 10X stock)
0.2 % BSA (fraction V)
Protease inhibitors (made up as 100X stock).
100 µg/ml bacitracin
100 µg/ml benzamidine
5 µg/ml aprotinin
5 µg/ml leupeptin

### Wash Buffer:

50 mM Hepes/10 mM MgCl₂, pH 7.4, ice cold (made from 10X stock).

### EXAMPLE 2: Estrogen Receptor Binding.

### Estrogen and estrogen agonist / antagonist binding affinity was measured by the following protocol:

cDNA cloning of human ERα: The coding region of human ERα was cloned by RT-PCR from human breast cancer cell mRNA using Expand™ High Fidelity PCR System according to manufacturer's instructions (Boehringer-Mannheim, Indianapolis, IN). PCR products were cloned into pCR2.1 TA Cloning Kit (Invitrogen, Carlsbad, CA) and sequenced. Each receptor-coding region was subcloned into the mammalian expression vector pcDNA3 ((Invitrogen, Carlsbad, CA).

Mammalian cell expression. Receptor proteins were overexpressed in 293T cells. These cells, derived from HEK293 cells (ATCC, Manassas, VA), have been engineered to stably express large T antigen and can therefore replicate plasmids containing a SV40 origin of replication to high copy numbers. 293T cells were transfected with either hERα-pcDNA3 or hERβ-pcDNA3 using lipofectamine as described by the manufacturer (Gibco/BRL, Bethesda, MD). Cells were harvested in phosphate buffered saline (PBS) with 0.5 mM EDTA at 48 h post-transfection. Cell pellets were washed once with PBS/EDTA. Whole cell lysates were prepared by homogenization in TEG buffer (50 mM Tris pH 7.4, 1.5 mM EDTA, 50 mM NaCI, 10% glycerol, 5 mM DTT, 5 µg/ml aprotinin, 10 µg/ml leupeptin, 0.1 mg/ml Pefabloc) using a dounce homogenizor. Extracts were centrifuged at 100,000 x g for 2 h at 4°C and supernatants were collected. Total protein concentrations were determined using BioRad reagent (BioRad, Hercules, CA).

Competition binding assay. The ability of various compounds to inhibit [³H]-estradiol binding was measured by a competition binding assay using dextran-coated charcoal as has been described (Leake RE, Habib F 1987 Steroid hormone receptors: assay and characterization. In: B. Green and R.E. Leake (eds). Steroid Hormones a Practical Approach. IRL Press Ltd, Oxford. 67-92.) 293T cell extracts expressing either hERα or hERβ were incubated in the presence of increasing concentrations of compound to be tested and a fixed concentration of [³H]-estradiol (141 µCi/mmol, New England Nuclear, Boston, MA) in 50 mM TrisHCl pH 7.4, 1.5 mM EDTA, 50 mM NaCI, 10% glycerol, 5 mM DTT, 0.5 mg/mL β-lactoglobulin in a final volume of 0.2 mL. All compounds to be tested were dissolved in dimethylsulfoxide. The final concentration of receptor was 50 pM with 0.5 nM [³H]-estradiol. After 16 h at 4°C, dextran-coated charcoal (20 µL) was added. After 15 min at room temperature the charcoal was removed by centrifugation and the radioactive ligand present in the supernatant was measured by scintillation counting. All reagents were obtained from Sigma (St. Louis, MO) unless otherwise indicated.

The binding affinity of (-)-cis-6-phenyl-5-[4-(2-pyrrolidin-1-yl-ethoxy)-phenyl]-5,6,7,8-tetrahydro-naphthalene-2-ol (PPTN) and 17β-estradiol were measured using recombinant human estrogen receptor (ER). Figure 1 shows the results of the binding experiment in which the binding of PPTN was found to be similar to that of 17β-estradiol.

### Example 3: Inhibition of In Vitro Human Breast Tumor Cell Growth.

The *in vitro* antiproliferative effects of (-)-cis-6-phenyl-5-[4-(2-pyrrolidin-1-yl-ethoxy)-phenyl]-5,6,7,8-tetrahydro-naphthalene-2-ol (PPTN) were tested using two types of human breast cancer cell lines: first, MCF-7 cells, which contain ER as well as progesterone receptors (PgR), and second, MDA-MB-231 cells, which lack ER and PgR, and enable the determination of an effect that is independent of the ER mechanism. The effect of PPTN on the growth of these different cell lines was determined by incubation of the cells with various compound concentrations for 6 days.

The antiproliferative effects were then determined by direct cell counts. PPTN inhibited the growth of the ER-positive cell line MCF-7. The IC₅₀ for growth inhibition was approximately 3 to 5 x 10⁻¹¹ M. In MDA-MB-231, ER-negative cell lines, the compound did not inhibit cell proliferation. These results demonstrate that growth inhibition was ER-specific and not due to cytotoxicity since the compound had no measurable effect on the ER-negative cell line.

### Example 4: Measurement of sexual functioning in post-menopausal women.

Sexual functioning and satisfaction in post-menopausal women is evaluated in a 52 week, placebo-controlled clinical study using a modified Women's Health Questionnaire (WHQ) as the measurement technique. Prior to the commencement in the study, post-menopausal women are divided into two groups of between 5 and 100 women in each group. One group is a placebo control group. The other group is a test group that receives a pharmaceutical composition containing an estrogen agonist / antagonist. At the start of the study, all participants in both groups complete a WHQ. Participants in the control group receive a daily placebo composition. Participants in the test group receive a composition containing an estrogen agonist / antagonist. At the end of the study, participants in both groups again complete the WHQ. The results of the WHQ from the control group and the test group are then compared.

The Women's Health Questionnaire (WHQ) provides a detailed examination of minor psychological and somatic symptoms experienced by peri- and postmenopausal women (Hunter M., et al., Maturitas; 8: 217, 1986). The WHQ is well documented in terms of reliability and validity. The questionnaire has 36 questions rated on four-point scales. The higher the score, the more pronounced is the distress and dysfunction. The 36 items combine into nine factors describing somatic symptoms, depressed mood, cognitive difficulties, anxiety/fear, sexual functioning, vasomotor symptoms, sleep problems, menstrual symptoms and attraction. The modified Woman's Health Questionnaire for this study contains specific questions regarding female sexual dysfunction including, for example, hypoactive sexual desire disorder, sexual arousal disorder, dyspareunia and vaginismus.

## Claims

1. Use of a compound that attenuates the binding of agouti-related protein to melanocortin receptors, but does not attenuate the binding of α-melanocyte stimulating hormone to melanocortin receptors, for the preparation of a medicament for the treatment of sexual arousal disorder in a female patient.

2. Use of a compound that attenuates the binding of agouti-related protein to melanocortin receptors, but does not attenuate the binding of α-melanocyte stimulating hormone to melanocortin receptors, for the preparation of a medicament for the treatment of vaginismus in a female patient.

3. Use of a compound that attenuates the binding of agouti-related protein to melanocortin receptors, but does not attenuate the binding of α-melanocyte stimulating hormone to melanocortin receptors, for the preparation of a medicament for increasing the frequency or intensity of orgasms in a female patient.

4. Use of a compound that attenuates the binding of agouti-related protein to melanocortin receptors, but does not attenuate the binding of α-melanocyte stimulating hormone to melanocortin receptors, for the preparation of a medicament for enhancing libido more than normal in a female patient.

5. The use of claim 1, 2, 3 or 4 wherein the melanocortin receptors are melanocortin-4 or melanocortin-3 receptors.

6. A pharmaceutical composition which comprises 1) a compound that attenuates the binding of agouti-related protein to melanocortin receptors, but does not attenuate the binding of α-melanocyte stimulating hormone to melanocortin receptors, and 2) an estrogen agonist / antagonist or a pharmaceutically acceptable salt thereof.

7. A pharmaceutical composition which comprises 1) a compound that attenuates the binding of agouti-related protein to melanocortin receptors, but does not attenuate the binding of α-melanocyte stimulating hormone to melanocortin receptors; 2) a compound that is a melanocortin receptor agonist; and 3) an estrogen agonist / antagonist or a pharmaceutically acceptable salt thereof.

8. A pharmaceutical composition of claim 6 or 7 wherein said estrogen agonist / antagonist is of the following formula (I): 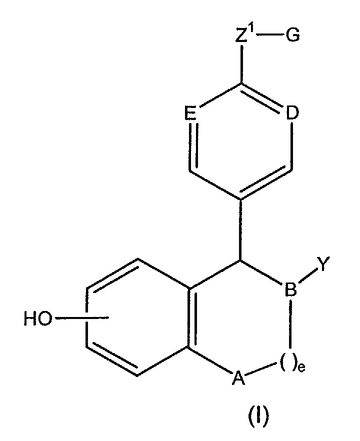 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚ W(CH₂)_{q}-;
(b) -O(CH₂)ₚ CR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 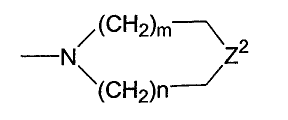 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 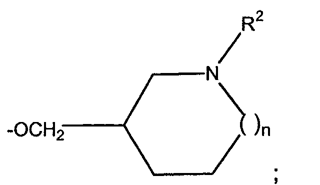
W is
(a) -CH₂-;
(b) -CH=CH-;
(c) -O-;
(d) -NR²-;
(e) -S(O)ₙ-; 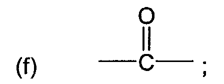
(g) -CR²(OH)-;
(h) -CONR²-;
(i) -NR²CO-;
(j) 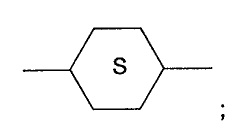 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxy (C₁-C₄)alkyl;
(j) aryl (C₁-C₄)alkyl;
(k) -CO₂H;
(I) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester, quaternary ammonium salt or prodrug thereof.

9. A composition of claim 8 wherein said estrogen agonist / antagonist is a compound of formula (IA): 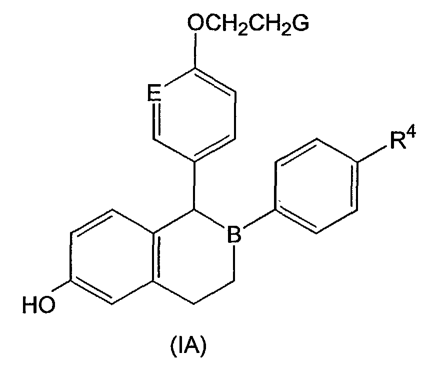 wherein
G is 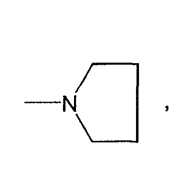 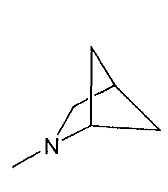 or 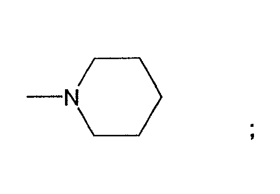
R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

10. A composition of claim 9 wherein said estrogen agonist / antagonist is (-)-cis-6-phenyl-5-[4-(2-pyrrolidin-1-yl-ethoxy)-phenyl]-5,6,7,8-tetrahydronaphthalene-2-ol or an optical or geometric isomer thereof; a pharmaceutically acceptable salt, N-oxide, ester, quaternary ammonium salt, or a prodrug thereof.

11. A composition of claim 10 wherein said estrogen agonist / antagonist is in the form of a D-tartrate salt.

12. A pharmaceutical composition for treating female sexual dysfunction which comprises 1) a compound that attenuates the binding of agouti-related protein to melanocortin receptors, but does not attenuate the binding of α-melanocyte stimulating hormone to melanocortin receptors, and 2) a compound selected from the group consisting of a cyclic guanosine 3',5'-monophosphate elevator.

13. A pharmaceutical composition of claim 12 wherein said cyclic guanosine 3',5'-monophosphate elevator is a PDE_{V} phosphodiesterase inhibitor.

14. A pharmaceutical composition of claim 13 wherein said PDE_{V} phosphodiesterase inhibitor is 1-[[3-(6,7-dihydro-1-methyl-7-oxo-3-propyl-1H-pyrazolo[4,3-d]pyrimidin-5-yl)-4-ethoxy-phenyl]sufonyl]-4-methylpiperazine citrate salt, 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one or 5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one.

15. A pharmaceutical composition which comprises 1) a compound that attenuates the binding of agouti-related protein to melanocortin receptors, but does not attenuate the binding of α-melanocyte stimulating hormone to melanocortin receptors, and 2) an estrogen optionally with a progestin.
